# EUROPEAN PATENT APPLICATION

(11) **EP 3 444 276 A1**
(43) Date of publication of application: **20.02.2019**
(21) Application number: 17186683.3
(22) Date of filing: 17.08.2017
(51) Int. Cl.: C07K 16/28, A61K 39/395, A61K 31/454, A61K 31/675, A61K 35/17, A61P 35/02, A61K 45/06

(54) **IMPROVED CANCER TREATMENT USING NK CELLS**

(71) Applicant: Onkocellular Limited, Donegal Town (IE)
(72) Inventor: The designation of the inventor has not yet been filed
(74) Representative: Schlich, George

(57) **Abstract**

Treatment of cancer in a patient comprises administering to the patient an antibody and a non-antibody agent that upregulates expression of Fc receptors on a cell of the cancer, wherein the antibody binds an antigen on the surface of the patient's own NK cells and the antibody Fc region binds to an Fc receptor on a cell of the cancer. Anticancer activity is via resultant killing action of the NK cell on the cancer cell, now binding the antibody Fc region, via reverse antibody-dependent cell-mediated cytotoxicity (R-ADCC).

## Description

### Introduction

The present invention relates to NK cells, antibodies and to related methods, uses and compositions for treatment of cancers.

### Background to the Invention

Acute myeloid leukemia (AML) is a hematopoietic malignancy involving precursor cells committed to myeloid development, and accounts for a significant proportion of acute leukemias in both adults (90%) and children (15-20%) (Hurwitz, Mounce et al. 1995; Lowenberg, Downing et al. 1999). Despite 80% of patients achieving remission with standard chemotherapy (Hurwitz, Mounce et al. 1995; Ribeiro, Razzouk et al. 2005), survival remains unsatisfactory because of high relapse rates from minimal residual disease (MRD). The five-year survival is age-dependent; 60% in children (Rubnitz 2012), 40% in adults under 65 (Lowenberg, Downing et al. 1999) and 10% in adults over 65 (Ferrara and Schiffer 2013). These outcomes can be improved if patients have a matched hematopoietic cell donor, but most do not, highlighting the need for an alternative approach to treatment.

Natural killer (NK) cells are cytotoxic lymphocytes, with distinct phenotypes and effector functions that differ from e.g. natural killer T (NK-T) cells. For example, while NK-T cells express both CD3 and T cell antigen receptors (TCRs), NK cells do not. NK cells are often found to express the markers CD16 and CD56, wherein CD16 functions as an Fc receptor and mediates antibody dependent cell-mediated cytotoxicity (ADCC) which is discussed below.

NK cells and NK cell precursors can be activated using specific agents that increase their proliferation, differentiation, migration and/or cytotoxicity. For example, whilst IL-2 is known to increase NK cell proliferation and cytotoxicity, IL-12 is known to increase NK cell migration by upregulating expression of adhesion protein L-selectin (Lehmann et al, 2012).

Other NK cell activating agents, such as the Thalidomide derivative Lenalidomide, are known to directly and indirectly increase NK cell cytotoxicity against certain cancers. The Lenalidomide-induced indirect activation of NK cells is thought to be IL-2 dependent, since it is known that thalidomide derivatives stimulate release of IL-2 and IFNy from other immune cells by downregulating SOCS1 (Reddy et al, 2008). With respect to an AML subtype known as 'deletion 5q', a previous study has shown that one 55 year old female patient responded positively to treatment with lenalidomide (Lancet, JE. et al. 2007). Furthermore, lenalidomide has been shown to enhance the ADCC effect of elotuzumab against multiple myeloma *in vitro* and in a mouse xenograft model (Balasa, B. et al. 2015).

Despite NK cells being naturally cytotoxic, NK cell lines with increased cytotoxicity have been developed. NK-92 and KHYG-1 represent two cell lines that have been researched extensively and show promise in cancer therapeutics (Swift et al. 2011; Swift et al. 2012).

KHYG-1 cells are known to be pre-activated. Unlike endogenous NK cells, KHYG-1 cells are polarized at all times, increasing their cytotoxicity and making them quicker to respond to external stimuli. NK-92 cells have a higher baseline cytotoxicity than KHYG-1 cells.

Furthermore, NK cells express both activating and inhibitory receptors on their surface. Upon binding of ligand to activating receptors, e.g. NKp30, signals are produced that give rise to a more cytotoxic NK phenotype. NKp30-mediated NK activation has been shown to increase IFNγ production (De Maria, A. et al. 2011) and result in increased killing of blood cancer cells (Muller et al. 2014). Moreover, WO 2005/009465 describes co-administration of NK cells with antibodies, specific for activating receptors on the NK cell surface, as a treatment for viral infections via ADCC.

In haplotype transplantation, the graft-versus-leukemia (GVL) effect is believed to be mediated by NK cells when there is a KIR receptor-ligand mismatch, which can lead to improved survival in the treatment of AML (Ruggeri, Capanni et al. 2002; Ruggeri, Mancusi et al. 2005). Further, rapid NK recovery is associated with better outcome and a stronger GVL effect in patients undergoing haplotype T-depleted hematopoietic cell transplantation (HCT) in AML (Savani, Mielke et al. 2007). Other trials have used haploidentical NK cells expanded ex vivo to treat AML in adults (Miller, Soignier et al. 2005) and children (Rubnitz, Inaba et al. 2010).

Several permanent NK cell lines have been established, and the most notable is NK-92 (mentioned above), derived from a patient with non-Hodgkin's lymphoma expressing typical NK cell markers except for CD16 (Fc gamma receptor). NK-92 has undergone extensive preclinical testing and exhibits superior lysis against a broad range of tumours compared with activated NK cells and lymphokine-activated killer (LAK) cells (Gong, Maki et al. 1994). Cytotoxicity of NK-92 cells against primary AML has been established (Yan, Steinherz et al. 1998). Another NK cell line, KHYG-1, derived from a patient with an aggressive NK cell leukemia, has been identified as a potential contender for clinical use (Suck et al. 2005) and retains its cytotoxicity when irradiated (Suck et al. 2006) but nevertheless has reduced cytotoxicity so has received less attention than NK-92.

ADCC is a well-known phenomenon in which NK cells recognize the Fc region of antibodies bound to target cells and promote target cell killing (Grier et al. 2012; Deng et al. 2014; Kobayashi et al. 2014). In order for this to occur, the NK cells must express Fc receptors (FcRs). Following binding of the NK FcRs to the Fc region of the antibodies, a more cytotoxic NK phenotype prevails. This often leads to increased killing of the target cells to which the antibody specifically binds.

Notter et al. demonstrated that precoating lymphokine activated killer (LAK) cells with anti-CD3 antibodies could enhance killing of autologous AML blasts. A combination of IL-2, IFN-γ and anti-CD3 monoclonal antibody was proposed as a potential treatment for AML.

However, all current adoptive immunotherapy protocols are affected by donor variability in the quantity and quality of effector cells, variables that could be eliminated if effective cell lines were available to provide more standardized therapy. T cells targeted to tumors is proposed as a therapy option, e.g. via chimeric antigen receptor (CAR) modifications, but this requires genetic manipulation of T cells.

In a previously published conference abstract (Williams, BA. et al. 2014), reverse antibody-dependent cell-mediated cytotoxicity (R-ADCC) is first introduced into the state of the art and it is proposed as a mechanism for treating AML. In WO2016/176756, published in November 2016 by the same inventors, it is proposed to treat leukemias and other blood cancers by administering NK cells and antibodies, killing cancer via R-ADCC.

There nevertheless exists a need for alternative and preferably improved treatments for leukemias, including AML, other blood cancers in general and still more generally other cancers of humans.

An object of the invention is to provide alternative methods, uses and compositions for treatment of tumours, especially cancers, especially in humans. Embodiments have as object the provision of improved methods, uses and compositions. More particular embodiments aim to provide treatments for identified cancers, e.g. blood cancers such as leukemias. Specific embodiments aim to take advantage of combinations of antibodies and effector cells in cancer therapies.

### Summary of the Invention

There are provided herein methods of treating cancer, using antibodies that bind to antigens on NK cells, wherein the combined complex of the antibodies with the NK cells then also binds to cancer cells and treats cancer via R-ADCC.

Also provided are the antibodies for use in such methods. The dogma in this field is that NK cells can have anti-tumourigenic properties that are antibody dependent, operating via antibody dependent cell-mediated cytotoxicity (ADCC). In this invention, while antibodies contribute to the NK cell-mediated killing of target cells, they do so via a different mechanism.

The invention provides cancer treatment comprising upregulating Fc receptor expression with an agent, wherein the agent is administered prior to, simultaneously with or subsequent to administering the antibody. The antibody binds the Fc receptor and also binds the NK cell and induces killing of cancer cells via R-ADCC.

According to the invention, there are provided methods of treating tumours, e.g. cancer, using a combination of the antibodies and agents capable of upregulating Fc receptor expression.

Compositions are provided comprising (a) an agent capable of upregulating Fc receptor expression, and (b) an antibody that binds to a particular antigen on NK cells. These are suitable for use in treatment of tumours and cancers via R-ADCC. The NK cells are suitably activated *ex vivo.*

There are also provided herein methods of treating cancer, using antibodies that bind to antigens found both on NK cells and also on cancers, wherein complexes of the antibodies with the NK cells then also bind to cancer cells and complexes of the antibodies with cancer cells then also bind to NK cells, and there is treatment of cancer via both R-ADCC and conventional ADCC occurring at the same time; optionally the NK cells are the patient's own (i.e. endogenous or autologous) NK cells. Also provided are the antibodies for use in such combined R-ADCC and ADCC methods.

Diseases particularly treatable according to the invention include cancers, blood cancers, leukemias, specifically acute myeloid leukemia and multiple myeloma. Tumours and cancers in humans in particular can be treated. References to tumours herein include references to neoplasms.

### Details of the Invention

As described in detail below in examples, treatment of tumour cells, specifically cancer cells, has been achieved using antibodies and agents capable of upregulating Fc receptor expression in combination, optionally also with NK cell activating agents. The inventors have shown utility of embodiments of the invention based on R-ADCC *in vitro* and using *in vivo* models of human cancer therapy.

According to the present invention there is therefore provided a method of treating a tumour in a patient, comprising administering an effective amount of an antibody to the patient, wherein the antibody binds an antigen on the surface of an endogenous natural killer (NK) cell and the antibody binds to an Fc receptor on a cell of the tumour.

In examples below, it is shown how the antibody may suitably bind an Fc receptor on the surface of the tumour cell, e.g. selected from CD16 (FcγRIII), CD32 (FcγII) or CD64 (FcγI). The antibody comprises an Fc region or otherwise a portion that is capable of binding an Fc receptor on a tumour cell. Ball *et al.* conducted a study of the expression of Fcγ receptors on primary AML and noted the following frequencies: FcγRI (58%); FcγII, (67%); and Fcγ III, (26%). FcγI and II receptor expression was highly correlated with FAB M4 and M5 morphology. Hence, treatments of the invention are suitable for tumours / cancers identified to express one or more Fc receptor.

In one example, a humanized or fully human anti-NK cell antibody is administered in combination with an effective amount of a chemotherapy agent.

Preferably, the chemotherapy agent upregulates expression of one or more Fc receptors on the cancer. Optionally, an agent capable of activating NK cells is also administered. Suitably, this antibody is specific for an NK activating receptor, e.g. anti-NKp30, anti-NKp44 or SLAMF7 (CS-1). The chemotherapy agent is preferably an alkylating agent and it may be administered separately from the antibody. The activating agent is preferably not an antibody and it may be administered separately from the antibody. In a preferred embodiment, the anti-NK cell antibody is Elotuzumab, the alkylating agent is cyclophosphamide and the activating agent is lenalidomide; dependent on the cancer being treated, this combination can be optionally supplemented with dexamethasone.

In embodiments, antibodies, according to the invention, are specific for NK inhibitory receptors e.g. KIR or CD137, wherein binding blocks receptor-ligand interaction and hence inhibitory signals.

Active agents of the invention for human use are produced in accordance with various regulations, e.g. under cGMP-compliant conditions. Conveniently, all of the active agents mentioned immediately above are FDA-approved.

Administration of the chemotherapy agent and/or activating agent can occur prior to, simultaneously with or subsequent to administration of the antibody.

Activation of an endogenous NK cell or an autologous NK cell occurs upon administration of the activating agent, wherein activation may comprise increasing one or more or all of NK proliferation, differentiation, migration and/or cytotoxicity.

In examples, the chemotherapy agent is an alkylating agent selected from a nitrogen mustard, a nitrosourea, an alkyl sulfonate, a triazine or an ethylenimine. nitrogen mustard. Preferably, the alkylating agent is a nitrogen mustard selected from bendamustine, chlorambucil, cyclophosphamide, ifosfamide, mechlorethamine and melphalan.

It is preferred that the chemotherapy agent is capable of upregulating Fc receptor expression on the cancer. Most preferably, the chemotherapy agent is cyclophosphamide.

As explained in examples below, a chemotherapy agent can be administered to a patient in need thereof at a low dose, in order to increase Fc receptor expression on cancer cells. This has the advantage that a number of side-effects associated with high dose chemotherapy are avoided, while retaining the beneficial effects of the invention.

In examples, the activating agent is selected from IL-2, IL-12, IL-15, a thalidomide derivative e.g. lenalidomide, pomalidomide or CC-122, a proteasome inhibitor, a histone deacetylase inhibitor, a selenium derivative and an activating antibody.

In a further preferred embodiment, the activating agent(s) is/are lenalidomide and/or IL-2. Lenalidomide has previously been shown to increase both the proportion of NK cells producing IFNγ and the amount of IFNγ produced per cell (Lagrue et al, 2015). Furthermore, Lagrue et al were able to show that lenalidomide lowers the activation dose of Rituximab (an anti-CD20 monoclonal antibody used for treating B cell malignancies via ADCC).

In another embodiment, the activating agent is an activating antibody specific for an antigen on the cancer cell surface and wherein binding of the antibody to its antigen indirectly activates endogenous NK cells, e.g. by blocking NK inhibitory receptor ligands on the surface of the cancer cell from binding inhibitory receptors (e.g. KIR or CD137) on the surface of the NK cell.

In further embodiments, the activating antibody is specific for an endogenous NK antigen.

Binding of the (R-ADCC) antibody to the endogenous NK cell and binding of that bound combination to a tumour cell leads to tumour cell death. Hence, a role of the antibody is in effect to cross-link the patient's own NK cell to the target cancer cell. Typically, the antibodies comprise an Fc region (which by definition binds Fc receptors). Other suitable antibodies may comprise portions that are not strictly speaking Fc regions (for whatever reason) but which nevertheless in use bind the Fc receptors on the tumour cells rather than NK cells. It is thus a feature of particular embodiments of the invention that the NK cell does not express an Fc receptor.

Antibodies may be used as a set or plurality of antibodies having substantially all the same binding properties. Alternatively, mixtures may be used of antibodies that bind to different NK cell surface markers or proteins or of antibodies containing different Fc regions, or in fact different antibodies may differ in both respects within a plurality of antibodies to be used in the treatment.

Killing of tumour cells is achieved in use of specific embodiments of the invention through a mechanism referred to as reverse antibody dependent cell-mediated cytotoxicity (R-ADCC). Specific examples included herein demonstrate this and are supported by control and verification examples that now plausibly show the tumour cell cytotoxicity as a result of R-ADCC.

Methods, uses and compositions herein described, above and below, are suitable for treatment of cancer, in particular cancer in humans, e.g. for treatment of cancers of blood cells or solid cancers.

In particular embodiments of the invention, killing of cancer cells comprises killing cancer stem cells, offering improved cancer therapy. Specific examples of the invention, set out below in more detail, demonstrate killing of clonogenic leukemia cancer cells. It is known that CD32-expressing quiescent leukemic stem cells (LSCs) are a major cause of relapse in AML following chemotherapy (Saito et al, 2010; Goff and Jamieson, 2010). The present invention provides a therapy capable of targeting quiescent cancer stem cells, e.g. LSCs, and hence is capable of lowering the chance of relapse in AML.

Embodiments of the invention are especially suitable for treatment of hematologic cancers, being cancers of the blood, bone marrow and/or lymph nodes. These include leukemias, lymphomas and myelomas. Specific cancers treatable are selected from acute lymphocytic leukemia (ALL), acute myeloid leukemia (AML), chronic lymphocytic leukemia (CLL) and chronic myeloid leukemia (CML), Hodgkin's lymphoma, non-Hodgkin's lymphomas, including T-cell lymphomas and B-cell lymphomas, asymptomatic myeloma, smoldering multiple myeloma (SMM), active myeloma and light chain myeloma . In particular, the invention is of use to treat ALL, AML, multiple myeloma and B-cell lymphomas.

Further embodiments of the invention are especially suitable for treatment of cancers characterized by expression of Fc receptors on the cancer cell surface. These include ALL, AML, myelomas, B-cell lymphomas and also melanoma, e.g. malignant melanoma.

Further specific examples of tumours treatable by the invention are selected from bladder carcinoma, chondrosarcoma, colorectal cancer, gastrointestinal cancer, glioma, head and neck cancer, kidney cancer, liver cancer, ovarian cancer, pancreatic cancer, soft tissue/muscle tissue cancer, prostate cancer, breast (mammary gland) cancer, germ cell cancer, multiple myeloma, histiocytic sarcoma, melanoma, skin cancer, uterine cancer and colon cancer.

Various routes of administration will be known to the skilled person to deliver active agents and combinations thereof to a patient in need. Embodiments of the invention are for blood cancer treatment. Administration of the antibody, or NK cells or combination thereof can be systemic or localized, such as for example via the intraperitoneal route

Activation of endogenous or autologous NK cells may suitably achieve homing of the NK cells to the tumour cells. Naturally, NK cells may not be found in large numbers in advanced tumours. This can be a result of tumours interfering with cytokine/chemokine signalling. Intratumoral cytokine/chemokine therapy can be used to target NK cells to cancers, e.g. IL-2, IL-12, IL-15 and IL-21 are all capable of activating endogenous NK cells at the tumour site and increasing lysis of the cancer cells (Zamai et al, 2007). Increased homing of NK effector cells to tumour sites may also be made possible by disruption of the tumour vasculature, e.g. by metronomic chemotherapy, or by using drugs targeting angiogenesis (Melero et al, 2014) to normalize NK cell infiltration via cancer blood vessels.

In other embodiments, an activating agent is administered more directly. Thus administration can be directly intratumoural, suitable especially for solid tumours. Administration can alternatively be intraperitoneal, such as in the case of metastatic ovarian cancer

Antibodies for use in the invention, whether in combination with a chemotherapy agent, an activating agent or in combination with activated exogenous NK cells, preferably bind to cell surface antigens, proteins or markers on the NK cells. Binding can attach the antibody leaving exposed, and separately available for binding, a portion that binds in turn to the tumour cells, via an Fc receptor. In examples, carried out and described herein, the antibody binds to an antigen/receptor on the NK surface. Suitable receptors are known NK cell activating receptors, e.g. natural cytotoxicity receptors. In use below, the antibodies activated or otherwise turned on (were agonists for) these activating receptors. As individual examples are NKp30, NKp44, NKp46, CS-1 and NKG2D.

In embodiments of the invention, activated NK cells are used in combination with an antibody specific for SLAMF7 (CS-1), e.g. to treat multiple myeloma (MM). The antigen is present on the NK cells, and hence treatment includes killing of target cells via R-ADCC. SLAMF7 antibodies are commercially available (e.g. Elotuzumab and others).

Other suitable antibodies for use in the invention include lirilumab (anti-KIR), urelumab (anti-CD137), ipilimumab (anti-CTLA-4) and nivolumab (anti-PD-1), subject to presence of the target antigen on the NK cell (or for particular embodiments described below both on NK cell and cancer).

In other specific embodiments of the invention, activated NK cells are used in combination with an antibody to treat AML. Known AML cells lack expression of SLAMF7 but do express CD32 (an Fc receptor). Therefore, ADCC using a SLAMF7-binding antibody is not possible, and treatment of AML occurs via R-ADCC.

In respect of the embodiments above and below, agents capable of increasing Fc receptor expression on cancer cells may be used. Suitable agents include a number of chemotherapy agents and all-trans retinoic acid (ATRA). Specifically, cyclophosphamide has been shown to increase expression of CD32 on myeloma cells, making the cells more sensitive to killing via R-ADCC therapy.

Of relevance to the embodiments described immediately above, and of general relevance to the invention as a whole, it is known from the prior art to use a specific monoclonal antibody therapy, targeted against SLAMF7, in combination with lenalidomide. The use was in ADCC therapy, and resulted in at most limited 'off-target' effects. Immune cell depletion was not a concern. Instead, that therapy offered a specific and efficient means to promote innate immunity and cancer killing (Liu et al, 2014).

In further embodiments, blocking antibodies are used, wherein the antibody binds and neutralizes an inhibitory receptor on the NK surface. Suitable antigens/receptors for the blocking antibodies include CD85d (LIR-2), CD85J (LIR-1), CD96 (TACTILE), CD152 (CTLA4), CD159a (NKG2A), CD223 (LAG-3), CD279 (PD-1), CD328 (SIGLEC7), SIGLEC9, TIGIT and TIM-3.

It is therefore preferred that the antibody is specific for an antigen expressed only on the surface of NK cells - the antigen is preferably not expressed on the cancer and preferably not expressed to a significant extent on any other cell. Nevertheless, if the activated autologous NK cells are pre-coated with antibody, prior to administration, this level of specificity is less important.

The invention provides a combination therapy, hence a method is provided herein for treating a tumour (especially a cancer) in a patient comprising administering to the patient an effective amount of the antibody in combination with an effective amount of a chemotherapy agent capable of upregulating Fc receptor expression on the tumour, wherein:
the antibody binds an antigen on the surface of NK cells in the patient;
the chemotherapy agent upregulates Fc receptor expression on the tumour;
the antibody binds to an Fc receptor on a cell of the tumour; and
the agent is not an antibody.

Optionally, the method further provides administering an agent capable of activating NK cells prior to, simultaneously with or subsequently to administering the antibody.

Alternatively, the invention provides a combination therapy for treating a tumour (especially a cancer) in a patient comprising administering to the patient an effective amount of an antibody, an effective amount of a chemotherapy agent capable of upregulating Fc receptor expression on the cancer and an effective amount of NK cells, wherein
the antibody binds an antigen on the surface of the NK cells;
the antibody binds to an Fc receptor on a cell of the cancer/tumour; and
the NK cells are exogenous (e.g. autologous or cell line derived) NK cells that have been activated *ex vivo.*

Administration of the NK cells is in combination with the antibody, so an effect of the two together is realized, and can occur prior to, simultaneously with or subsequent to administering the antibody. In an example, activated autologous NK cells are administered in combination with a humanized anti-NK activating receptor antibody, preferably anti-NKp30.

In carrying out the invention, treatment has been successful in which the antibody is administered bound to the NK cell. Pre-incubating the antibody with the NK cell prior to administering the antibody bound to the NK cell is one way to prepare this combination. In yet another example, the NK cells are pre-treated with a humanized anti-NK activating receptor antibody, preferably anti-NKp30, before administration, the NK cells having had their proliferation capacity diminished, e.g. by irradiation.

A patient's own NK cells in general are believed to be suitable for the methods, uses and compositions of the invention. Activated autologous NK cells are preferably used. Antibodies are utilized that bind the NK cell to be used, and it is believed that for all proposes NK cells, antibodies that bind cell surface molecules, preferably as described herein, can be identified.

In a specific embodiment of the invention, the activated NK cells administered to the patient are in the form of an NK cell line. These NK cell lines are optionally modified to have increased cytotoxicity against cancer cells.

The method of treating a tumour in a patient can comprise administering an effective amount of an activated autologous NK cell to the patient, wherein an antibody previously administered to the patient binds an antigen on the surface of the NK cell and the antibody further binds to an Fc receptor on a cell of the tumour. Preferred and optional aspects of earlier methods of the invention form preferred and optional aspects of these methods.

Still further, the invention provides the described antibodies, NK cells and combinations thereof for uses described herein. Thus the invention provides an antibody for use in treating a tumour in a patient, wherein the antibody binds an antigen on the surface of an endogenous NK cell and the antibody binds to an Fc receptor on a cell of the tumour. The invention provides an antibody and a chemotherapy agent for use in combination for treating a tumour in a patient, wherein the antibody binds an antigen on the surface of the NK cell and the antibody binds to an Fc receptor on a cell of the tumour. The invention also provides an activated autologous NK cell or NK cell line for use in treating a tumour in a patient, wherein an antibody administered to the patient binds an antigen on the surface of the NK cell and the antibody binds to an Fc receptor on a cell of the tumour.

The invention provides a composition comprising (a) a chemotherapy agent capable of increasing Fc receptor expression on a cancer, and (b) an antibody that binds NK cells. Preferably, the antibody activates NK cells. The composition is for use as described elsewhere herein, and thus is suitable, for treatment of tumours, e.g. tumours of blood cells or solid tumours. In specific embodiments the composition is for treatment of acute myeloid leukemia and/or multiple myeloma.

Still further, the invention provides a composition comprising (a) an activated autologous NK cell or NK cell line, (b) an antibody that binds to the NK cell, and (c) a chemotherapy agent capable of increasing Fc receptor expression on a cancer. The composition is for use as described elsewhere herein, and thus is suitable, for treatment of tumours, e.g. tumours of blood cells or solid tumours, suitable for treatment of cancers. In specific embodiments the composition is for treatment of leukemia, including acute myeloid leukemia, and myeloma, including multiple myeloma.

Antibodies in the compositions are typically as elsewhere described, and hence may bind an Fc receptor selected from CD16, CD32 or CD64, and may bind a NK cell cytotoxicity receptor, e.g. NKp30 and NKp44. Other features of the antibodies are as described in relation to methods and uses of the invention, and are not repeated here.

The invention still further provides methods and uses for treating cancer that combine ADCC-based approaches and R-ADCC approaches in the same therapy. In these embodiments, an antibody is chosen that is capable of binding to antigen found both on an NK cell and also on the cancer. This can also be described the other way round, in that these embodiments of the invention may comprise identifying an antigen found both on the cancer and also on NK cells of the patient (or on NK cells introduced as part of the therapy), and then selecting an antibody that binds to that common antigen. At the same time, the cancer is chosen as one that expresses one or more Fc receptors. The NK cells are endogenous / autologous and express one or more Fc receptors, or are exogenous and chosen so as to express one or more Fc receptors - noting that some NK cell lines identified as useful in cancer therapy do not express Fc receptors; such would not be suitable for these particular embodiments.

Treatment of cancer in a patient then comprises administering to the patient an antibody, wherein the antibody binds an antigen found both on NK cells used in the therapy and also on the cancer, and wherein the antibody Fc region binds during the therapy to an Fc receptor on a cell on an NK cell or alternatively an Fc receptor on the cancer. Any individual antibody has these binding capabilities but does not perform all simultaneously. Instead, during therapy, some antibodies bind antigen on the cancer and have their Fc portion bound to Fc receptors on NK cells (ADCC) while some antibodies bind antigen on NK cells and have their Fc portion bound to Fc receptors on the cancer (R-ADCC). Anticancer activity is via resultant killing action of the NK cell on the cancer cell, via both antibody-dependent cell-mediated cytotoxicity (ADCC) effected by some antibodies and reverse antibody-dependent cell-mediated cytotoxicity (R-ADCC) effected by others.

Accordingly, the invention provides an antibody for use in treating a cancer in a patient via a combination of both antibody-dependent cell-mediated cytotoxicity (ADCC) and reverse antibody-dependent cell-mediated cytotoxicity (R-ADCC), wherein
the antibody is capable of binding to an antigen found both on the surface of an NK cell and also on a cell of the cancer; and
the antibody further is capable of binding an Fc receptor on a cell of the cancer and also an Fc receptor on the NK cell.

In general, binding of antibodies to Fc receptors on NK cells is a known phenomenon. As described elsewhere herein the realization that Fc receptors on cancers may be targeted for an anti-cancer therapy is a feature of other embodiments of the invention. In these particular embodiments, there is an additional element, namely an antigen common to the cancer and the NK cell - which may be endogenous / autologous or exogenous. Because the antibody targets both the cancer and the NK cells, and because Fc receptors are present on cancer and NK cells, ADCC and R-ADCC can take place simultaneously in cancer treatment.

As in other therapies described herein, the antibody may be combined with an agent that is capable of activating endogenous or autologous NK cells. Optional and preferred features of embodiments described above also apply to these particular embodiments - as an example, the agent may be other than an antibody, e.g. can be a small molecule as described above such as a thalidomide derivative, e.g. lenalidomide. As a further example, Fc receptor expression on the cancer may be upregulated, e.g. by administration of all-trans retinoic acid. Still further, the treating may comprise administering the antibody alone or bound to a therapeutic NK cell.

In a specific example of these particular embodiments, the antibody binds to SLAMF7, e.g. is elotuzumab, as SLAMF7 has been identified as present both on certain NK cells and certain cancers.

In a further specific example of these particular embodiments, the antibody binds to SLAMF3, aka CD229, as SLAMF3 has been identified as present both on certain NK cells and certain cancers. Other antibodies specified herein form other preferred embodiments where the antigen they target is found both on cancer and NK cells.

These particular embodiments are suitable especially for treating blood cancer, including AML.

In a specific example of this particular series of embodiments, the invention provides a combination of elotuzumab, lenalidomide and cyclophosphamide for use in treating acute myeloid leukemia (AML) in a patient via R-ADCC, wherein
elotuzumab is capable of binding SLAMF7 on an NK cell;
elotuzumab further is capable of binding an Fc receptor on an AML cell;
cyclophosphamide is capable of upregulating expression of Fc receptors on an AML cell; and
lenalidomide further activates NK cells.

In a further specific example of this particular series of embodiments, the invention provides a combination of elotuzumab and cyclophosphamide for use in treating multiple myeloma (MM) in a patient via R-ADCC, wherein
elotuzumab is capable of binding SLAMF7 on an NK cell and on an MM cell;
elotuzumab further is capable of binding an Fc receptor on an MM cell and an Fc receptor on an NK cell; and
cyclophosphamide is capable of upregulating expression of Fc receptors on an MM cell.

A method of treating cancer is similarly provided by the invention, comprising administering an antibody to a patient, wherein
the antibody is capable of binding to an antigen found both on the surface of an NK cell and also on a cell of the cancer; and
the antibody further is capable of binding an Fc receptor on a cell of the cancer and also an Fc receptor on the NK cell
wherein cancer treatment is achieved via a combination of both antibody-dependent cell-mediated cytotoxicity (ADCC) and reverse antibody-dependent cell-mediated cytotoxicity (R-ADCC), and wherein Fc receptor expression is upregulated using a chemotherapy agent.

The NK cell may be an endogenous or autologous NK cell. The NK cell may also be an NK cell line. The treating may comprise administering the antibody bound to the NK cell. Other optional and preferred features of the invention as described above apply to these methods.

A method of establishing whether a cancer in a patient is treatable using the invention is also provided. The method comprises screening one or a plurality of cancer cells isolated from the patient for Fc receptor expression. Typically, the method comprises comparing the Fc receptor expression against a predetermined threshold, wherein an expression level at or above the threshold indicates the patient is treatable.

In use of the method, a sample of cancer cells is isolated from the patient and screened for Fc receptor (e.g. CD16, CD32 and CD64) expression using an *in vitro* assay, in order to determine the level of Fc receptor expression on the cancer cells and hence establish whether the cancer is treatable via R-ADCC. Expression levels can be indicated as % positive, whether expression is bright, intermediate or dim, or a combination of these. Threshold expressions levels for effective therapy are generally determined empirically, though it is believed that cancers wherein 20% or more, preferably 30% or more, cells assayed are positive for FcR expression are suitable for treatment according to the invention. The cancer cells assayed may be a sub-population of a larger population comprising cancer cells; cancer-propagating cells are a suitable sub-population though these make up a fraction, only e.g. 10% or less, of the total cancer cell population. Nevertheless, expression levels of Fc receptors on cancer cells can be increased using chemotherapy agents according the invention.

In embodiments, the *in vitro* assay utilizes labelled monoclonal antibodies, specific to Fc receptors, and Fc receptor expression is detected using flow cytometry. The threshold level of Fc receptor expression is suitably then determined by establishing a cut-off point at which the potential benefits of treatment via R-ADCC outweigh the risks associated with treatment.

In further embodiments, the Fc receptor expression level of a given cancer may be upregulated, in order for treatment according to the invention to be possible or improved. In a preferred embodiment, all-trans retinoic acid (ATRA) is used to upregulate Fc receptor expression on the cancer. ATRA is an FDA-approved chemotherapeutic agent for the treatment of acute promyelocytic leukemia. Methods and uses of the invention may thus comprise administration/use of an agent to upregulate Fc receptor expression. Optionally, the agent is a chemotherapy agent. Preferably, the chemotherapy agent is cyclophosphamide.

A further method of diagnosis is provided by the invention, comprising analyzing a sample from a patient having or suspected of having cancer to determine presence on one or more cancer cells of an antigen, and determining whether NK cells of the patient also express the same antigen. A preferred next step is then to determine if the cancer cells also express Fc receptors.

The results can suitably be used to identify patients who may benefit from the combined approach. Hence, presence of the same antigen on both cancer cells and NK cells may be used to indicate the patient is suitable for treatment by a therapy that combines ADCC and R-ADCC. As a specific example, if a cancer is noted to express SLAMF7 and Fc receptors, and patient's NK cells express SLAMF7 then treatment using antibodies to SLAMF7 is indicated as a potential therapy.

The invention as described herein provides the following embodiments.

### Embodiments relating to treatment using an antibody and an NK enhancing agent.

Embodiment 1. A method of treating a cancer in a patient via reverse antibody dependent cell-mediated cytotoxicity (R-ADCC), comprising administering to the patient an effective amount of an antibody in combination with an effective amount of an agent capable of activating endogenous natural killer (NK) cells, wherein the antibody binds an antigen on the surface of an endogenous NK cell; and the antibody binds an Fc receptor on a cell of the cancer.
Embodiment 2. A method according to embodiment 1, wherein the antibody binds an Fc receptor selected from CD16, CD32 or CD64.
Embodiment 3. A method according to embodiment 1 or 2, comprising administering the agent prior to, simultaneously with or subsequent to administering the antibody.
Embodiment 4. A method according to embodiments 1-3, wherein the activating comprises one or more or all of increasing NK proliferation, differentiation, migration and/or cytotoxicity.
Embodiment 5. A method according to embodiments 1-4, wherein the agent is selected from IL-2, IL-12, IL-15, a thalidomide derivative, a proteasome inhibitor, a histone deacetylase inhibitor, a selenium derivative or an activating antibody.
Embodiment 6. A method according to embodiment 5, wherein the activating antibody is specific for a cell of the cancer and indirectly activates endogenous NK cells.
Embodiment 7. A method according to embodiment 5 or 6, wherein the activating antibody binds an NK inhibitory receptor ligand on the surface of the cancer cell.
Embodiment 8. A method according to embodiment 5, wherein the activating antibody is specific for an endogenous NK antigen.
Embodiment 9. A method according to embodiment 5 or 8, wherein the activating antibody activates an activating receptor on the NK surface or blocks an inhibitory receptor on the NK surface.
Embodiment 10. A method according to any preceding embodiments, for treatment of cancer by killing cancer stem cells.
Embodiment 11. A method according to any preceding embodiments, for treatment of blood cancer.
Embodiment 12. A method according to any preceding embodiments, for treatment of leukemia, e.g. acute myeloid leukemia or acute lymphocytic leukemia.
Embodiment 13. A method according to any of embodiments 1 to 11, for treatment of B-cell lymphoma.
Embodiment 14. A method according to any of embodiments 1 to 10, for treatment of solid cancers.
Embodiment 15. A method according to any preceding embodiments, wherein the antibody binds to a NK cell surface marker, e.g. a cytotoxicity receptor, such as an antigen selected from NKp30 and NKp44.

### Embodiments relating to treatment using antibody and activated autologous NK cells

Embodiment 16. A method for treating a tumour in a patient via R-ADCC, comprising administering to the patient an effective amount of an antibody and an effective amount of NK cells, wherein
   the antibody binds an antigen on the surface of the NK cells;
   the antibody binds an Fc receptor on a cell of the tumour; and
   the NK cells are autologous NK cells that have been activated *ex vivo.*
Embodiment 17. A method according to embodiment 16, wherein activating the autologous NK cells comprises increasing one or more or all of NK proliferation, differentiation, migration and/or cytotoxicity.
Embodiment 18. A method according to embodiment 16 or 17, wherein activating the autologous NK cells *ex vivo* is achieved by treating the autologous NK cells with an effective amount of an agent.
Embodiment 19. A method according to embodiments 16-18, wherein the agent is selected from IL-2, IL-12, IL-15, a thalidomide derivative, a proteasome inhibitor, a histone deacetylase inhibitor, a selenium derivative and an activating antibody.
Embodiment 20. A method according to embodiment 19, wherein the activating antibody is specific for a cell of the cancer and indirectly activates endogenous NK cells.
Embodiment 21. A method according to embodiment 19 or 20, wherein the activating antibody binds an NK inhibitory receptor ligand on the surface of the tumour.
Embodiment 22. A method according to embodiment 19, wherein the activating antibody is specific for an endogenous NK antigen.
Embodiment 23. A method according to embodiment 19 or 22, wherein the activating antibody activates an activating receptor on the NK surface or blocks an inhibitory receptor on the NK surface.
Embodiment 24. A method according to embodiments 16-23, comprising administering the NK cells prior to, simultaneously with or subsequent to administering the antibody.
Embodiment 25. A method according to embodiments 16-23, comprising administering the antibody bound to the NK cell.
Embodiment 26. A method according to embodiment 25, comprising pre-incubating the antibody with the NK cell prior to administering the antibody bound to the NK cell.
Embodiment 27. A method according to any of embodiments 16 to 26, for treatment of tumours of blood cells or solid tumours.
Embodiment 28. A method according to any of embodiments 16 to 27, for treatment of leukemia.
Embodiment 29. A method according to embodiment 28, for treatment of acute myeloid leukemia.

### Embodiments relating to use in treatment

Embodiment 30. A combination of an antibody and an agent capable of activating endogenous natural killer (NK) cells, for use in treating a tumour in a patient via R-ADCC, wherein
   the antibody binds an antigen on the surface of a natural killer (NK) cell; and the antibody binds an Fc receptor on a cell of the tumour.
Embodiment 31. A combination for use according to embodiment 30, wherein the antibody binds an Fc receptor selected from CD16, CD32 or CD64.
Embodiment 32. A combination for use according to embodiment 30 or 31, comprising administering the agent prior to, simultaneously with or subsequent to administering the antibody.
Embodiment 33. A combination for use according to embodiments 30-32, wherein the agent is capable of activating the endogenous NK cells by increasing one or more or all of NK proliferation, differentiation, migration and/or cytotoxicity.
Embodiment 34. A combination for use according to embodiments 30-33, wherein the agent is selected from IL-2, IL-12, IL-15, a thalidomide derivative, a proteasome inhibitor, a histone deacetylase inhibitor, a selenium derivative or an activating antibody.
Embodiment 35. A combination for use according to embodiment 34, wherein the activating antibody is specific for a cell of the cancer and indirectly activates endogenous NK cells.
Embodiment 36. A combination for use according to embodiment 34 or 35, wherein the activating antibody binds an NK inhibitory receptor ligand on the surface of the cancer cell.
Embodiment 37. A combination for use according to embodiment 34, wherein the activating antibody is specific for an endogenous NK antigen.
Embodiment 38. A combination for use according to embodiment 34 or 37, wherein the activating antibody activates an activating receptor on the NK surface or blocks an inhibitory receptor on the NK surface.
Embodiment 39. A combination for use according to any of embodiments 30-38, for treatment of leukemia.
Embodiment 40. A combination of an antibody and a NK cell for use in combination for treating a tumour in a patient via R-ADCC, wherein
   the antibody binds an antigen on the surface of the NK cell;
   the antibody binds an Fc receptor on a cell of the tumour; and
   the NK cells are autologous NK cells that have been activated *ex vivo.*
Embodiment 41. A combination for use according to embodiment 40, wherein the autologous NK cells have been activated by increasing one or more or all of NK proliferation, differentiation, migration and/or cytotoxicity.
Embodiment 42. A combination for use according to embodiment 40 or 41, wherein activating the autologous NK cells *ex vivo* is achieved by treating the autologous NK cells with an effective amount of an agent.
Embodiment 43. A combination for use according to embodiments 40-42, wherein the agent is selected from IL-2, IL-12, IL-15, a thalidomide derivative, a proteasome inhibitor, a histone deacetylase inhibitor, a selenium derivative or an activating antibody.
Embodiment 44. A combination for use according to embodiment 43, wherein the activating antibody is specific for a cell of the cancer and indirectly activates endogenous NK cells.
Embodiment 45. A combination for use according to embodiment 43 or 44, wherein the activating antibody binds an NK inhibitory receptor ligand on the surface of the cancer cell.
Embodiment 46. A combination for use according to embodiment 43, wherein the activating antibody is specific for an endogenous NK antigen.
Embodiment 47. A combination for use according to embodiment 43 or 46, wherein the activating antibody activates an activating receptor on the NK surface or blocks an inhibitory receptor on the NK surface.
Embodiment 48. A combination for use according to embodiments 40-47, wherein the treatment comprises administering the NK cells prior to, simultaneously with or subsequent to administering the antibody.
Embodiment 49. A combination for use according to embodiment 48, wherein the treatment comprises administering the antibody bound to the NK cell.
Embodiment 50. A combination for use according to embodiment 49, comprising pre-incubating the antibody with the NK cell prior to administering the antibody bound to the NK cell.

### Embodiments relating to Compositions per se

Embodiment 51. A composition for treatment of a tumour in a patient via R-ADCC comprising:
   (a) a NK cell, and
   (b) an antibody that binds to the NK cell,
      wherein the NK cells are autologous NK cells that have been activated *ex vivo.*
Embodiment 52. A composition according to embodiment 51, wherein activating the autologous NK cells comprises one or more or all of increasing NK proliferation, differentiation, migration and/or cytotoxicity.
Embodiment 53. A composition according to 51 or 52, wherein activating the autologous NK cells *ex vivo* is achieved by treating the autologous NK cells with an effective amount of an agent.
Embodiment 54. A composition according to embodiments 51-53, wherein the agent is selected from IL-2, IL-12, IL-15, a thalidomide derivative, a proteasome inhibitor, a histone deacetylase inhibitor, a selenium derivative or an activating antibody.
Embodiment 55. A composition according to embodiment 54, wherein the activating antibody is specific for a cell of the cancer and indirectly activates endogenous NK cells.
Embodiment 56. A composition according to embodiment 54 or 55, wherein the activating antibody binds an NK inhibitory receptor ligand on the surface of the cancer cell.
Embodiment 57. A composition according to embodiment 54, wherein the activating antibody is specific for an endogenous NK antigen.
Embodiment 58. A composition according to embodiment 54 or 57, wherein the activating antibody activates an activating receptor on the NK surface or blocks an inhibitory receptor on the NK surface.
Embodiment 59. A composition according to embodiments 51-58, for treatment of tumours of blood cells or solid tumours.
Embodiment 60. A composition according to embodiments 51-59, for treatment of leukemia.
Embodiment 61. A composition according to embodiments 51-59, for treatment of acute myeloid leukemia.
Embodiment 62. A composition according to embodiments 51-61, wherein the antibody binds an Fc receptor selected from CD16, CD32 or CD64.
Embodiment 63. A composition according to embodiments 51-62, wherein the antibody binds to a cytotoxicity receptor, e.g. a NK cell surface antigen selected from NKp30 and NKp44.

### Embodiments relating to Assays

Embodiment 64. A method of establishing whether a cancer in a patient is treatable using R-ADCC, wherein the method comprises screening one or a plurality of cancer cells isolated from the patient for Fc receptor expression.
Embodiment 65. A method according to embodiment 64, comprising comparing the Fc receptor expression against a predetermined threshold, wherein an expression level at or above the threshold indicates the patient is treatable.
Embodiment 66. A method according to embodiment 65, wherein the threshold Fc receptor expression is determined by establishing a cut-off point at which the potential benefits of treatment via R-ADCC outweigh the risks associated with treatment.
Embodiment 67. A method according to any of embodiments 64 to 66, wherein the Fc receptor is selected from one or more or all of CD16, CD32 and CD64.
Embodiment 68. A method according to any of embodiments 64 to 67, wherein the screening utilizes labelled monoclonal antibodies, specific to Fc receptors, and wherein binding of said antibodies is detected using flow cytometry.

Similarly, other optional and preferred features of the NK cells of the composition are as described elsewhere in relation to other methods and uses of the invention.

### Embodiments relating to upregulation of Fc receptor expression

A1. A combination of an antibody and a chemotherapy agent, for use in treating a cancer expressing Fc receptors in a patient via reverse antibody-dependent cell-mediated cytotoxicity (R-ADCC), wherein
   the antibody binds an antigen on the surface of an endogenous or autologous natural killer (NK) cell;
   the antibody further binds an Fc receptor on a cell of the cancer; and
   the agent is not an antibody.
A2. A combination for use according to embodiment A1, further comprising an agent capable of activating endogenous or autologous NK cells.
A3. A combination for use according to either embodiment A1 or A2, wherein the autologous NK cell is expanded and activated ex vivo and administered to the patient in combination with the antibody and chemotherapy agent.
A4. A combination for use according to embodiment A3, wherein the treating comprises administering the antibody bound to the NK cell.
A5. A combination for use according to any of embodiments A2 to A4, wherein the activating agent is a thalidomide derivative, e.g. lenalidomide.
A6. A combination for use according to any preceding embodiment, wherein the treating comprises upregulating Fc receptor expression on the cancer, e.g. by administration of the chemotherapy agent.
A7. A combination for use according to any preceding embodiment, wherein the chemotherapy agent is an alkylating agent.
A8. A combination for use according to embodiment A7, wherein the alkylating agent is selected from a nitrogen mustard, a nitrosourea, an alkyl sulfonate, a triazine or an ethylenimine.
A9. A combination for use according to embodiment A8, wherein the nitrogen mustard is selected from bendamustine, chlorambucil, cyclophosphamide, ifosfamide, mechlorethamine or melphalan.
A10. A combination for use according to embodiment A9, wherein the nitrogen mustard is cyclophosphamide.
A11. A combination for use according to any preceding embodiment, wherein the cancer does not express an antigen for the antibody.
A12. A combination for use according to any preceding embodiment, wherein the cancer is blood cancer.
A13. A combination for use according to embodiment A12, wherein the blood cancer is selected from acute lymphocytic leukemia (ALL), acute myeloid leukemia (AML), chronic lymphocytic leukemia (CLL), chronic myeloid leukemia (CML), Hodgkin's lymphoma, non-Hodgkin's lymphoma, including T-cell lymphomas and B-cell lymphomas, asymptomatic myeloma, smoldering multiple myeloma (SMM), active myeloma or light chain myeloma.
A14. A combination for use according to either embodiment A12 or A13, wherein the blood cancer is AML.
A15. A combination for use according to either embodiment A12 or A13, wherein the blood cancer is multiple myeloma.
A16. A composition for the treatment of cancer, comprising the combination of an antibody and a chemotherapy agent according to any preceding embodiment.
A17. An in vitro assay for establishing whether a cancer in a patient is treatable using R-ADCC, wherein the assay comprises screening one or a plurality of cancer cells isolated from the patient for Fc receptor expression.
A18. An in vitro assay according to embodiment A17, wherein a positive result indicates that the patient is treatable with a combination for use according to any of embodiments A1 to A15 or a composition according to embodiment A16.
A19. A method of treating a cancer expressing Fc receptors in a patient via R-ADCC, comprising administering to the patient an effective amount of an antibody in combination with an effective amount of a chemotherapy agent capable of upregulating Fc receptor expression on the cancer, wherein
   the antibody binds an antigen on the surface of an endogenous NK cell;
   the antibody binds an Fc receptor on a cell of the cancer; and
   the agent is not an antibody.
A20. A method according to embodiment A19, further comprising administering an agent capable of activating NK cells prior to, simultaneously with or subsequently to administering the antibody.
A21. A method according to embodiment A20, wherein the activating agent is lenalidomide.
A22. A method according to any of embodiments A19 to A21, wherein the chemotherapy agent is an alkylating agent.
A23. A method according to embodiment A22, wherein the alkylating agent is selected from a nitrogen mustard, a nitrosourea, an alkyl sulfonate, a triazine or an ethylenimine.
A24. A method according to embodiment A23, wherein the nitrogen mustard is selected from bendamustine, chlorambucil, cyclophosphamide, ifosfamide, mechlorethamine or melphalan.
A25. A method according to embodiment A24, wherein the nitrogen mustard is cyclophosphamide.
A26. A method according to any of embodiments A19 to A25, wherein the cancer does not express an antigen for the antibody.
A27. A method according to any of embodiments A19 to A26, wherein the cancer is blood cancer.
A28. A method according to embodiment A27, wherein the blood cancer is selected from acute lymphocytic leukemia (ALL), acute myeloid leukemia (AML), chronic lymphocytic leukemia (CLL), chronic myeloid leukemia (CML), Hodgkin's lymphoma, non-Hodgkin's lymphoma, including T-cell lymphomas and B-cell lymphomas, asymptomatic myeloma, smoldering multiple myeloma (SMM), active myeloma or light chain myeloma.
A29. A method according to either embodiment A27 or A28, wherein the blood cancer is AML.
A30. A method according to either embodiment A27 or A28, wherein the blood cancer is multiple myeloma.
A31. A method of establishing whether a cancer in a patient is treatable using R-ADCC, wherein the method comprises screening one or a plurality of cancer cells isolated from the patient for Fc receptor expression.
A32. A method according to embodiment A31, wherein the screening utilizes labelled monoclonal antibodies, specific to Fc receptors, and wherein binding of said antibodies is detected using flow cytometry.

### Examples

The present invention is now described in more and specific details in relation to the use of activated endogenous or autologous NK cells for treating blood cancer via R-ADCC. The invention is illustrated in the following examples, with reference to the accompanying drawings, in which:
Fig. 1 shows NK-92 and KHYG-1 cytotoxicity against a panel of leukemia cell lines;
Fig. 2 shows the effect of antibody pre-treatment of activating receptors on KHYG-1 cytotoxicity against leukemia cell lines;
Fig. 3 shows the effect of antibody pre-treatment of activating receptors on KHYG-1 cytotoxicity against primary AML samples;
Fig. 4 shows the effect of antibody pre-treatment with varying concentrations of anti-NKp30 and anti-NKp44 on KHYG-1 cytotoxicity against leukemia cell lines;

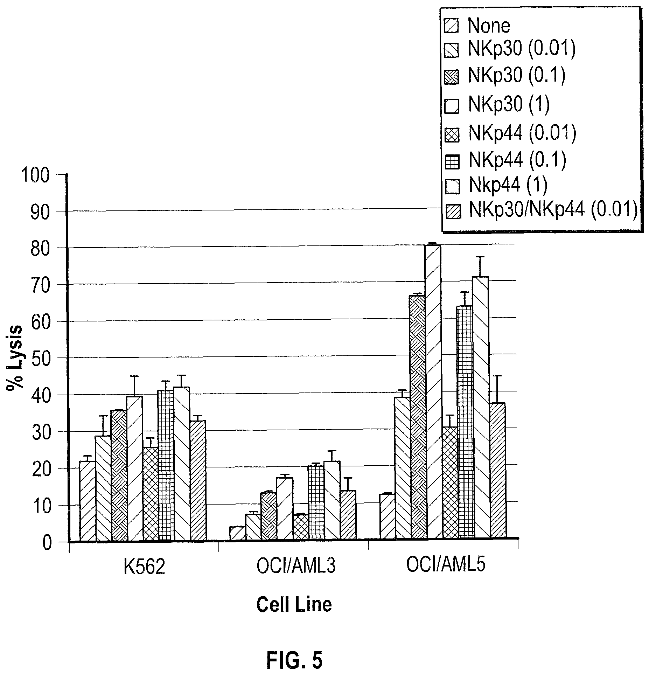

Fig. 5 shows the effect of antibody pre-treatment with varying concentrations of anti-NKp30 and anti-NKp44 on KHYG-1 cytotoxicity against leukemia cell lines;

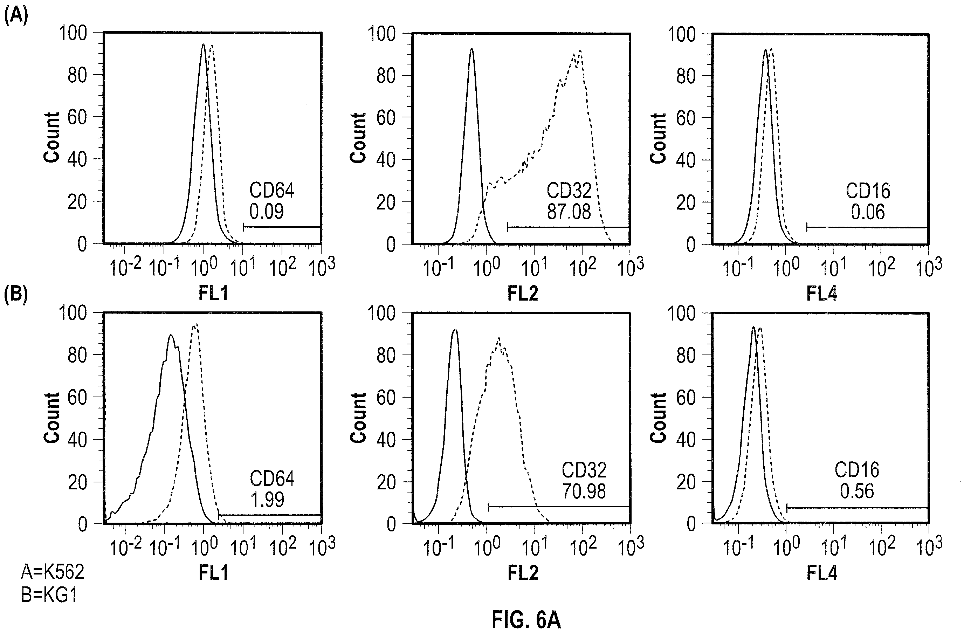

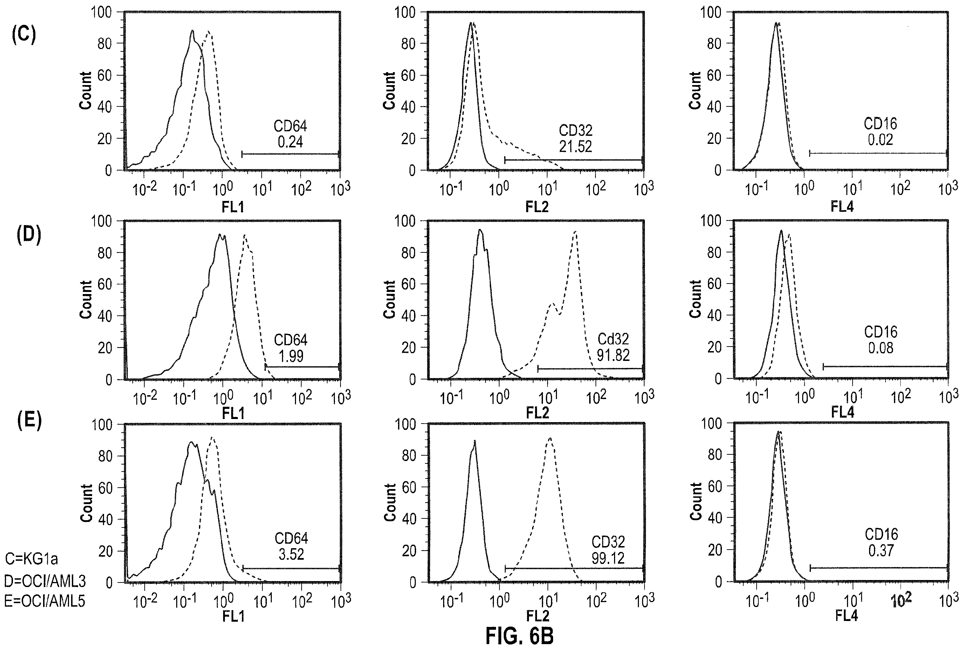

Fig. 6 shows Fc gamma receptor expression on leukemia cell lines (K562, KG1, KG1a, OCI/AML3, OCI/AML5);

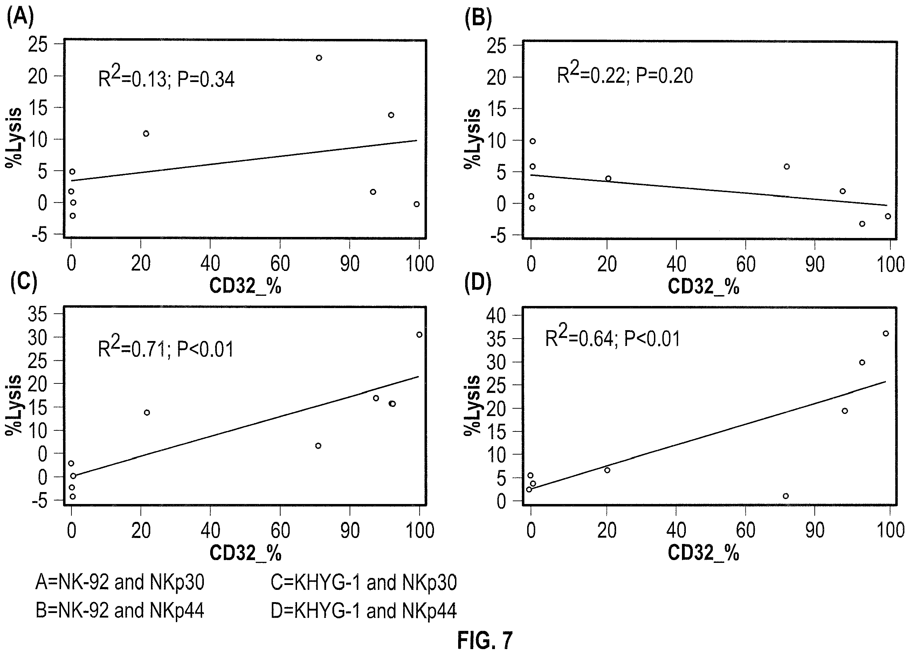

Fig. 7 shows regression analysis of CD32 expression and delta cytotoxicity of NKp30 and NKp44 pretreated NK-92 and KHYG-1 cells;
Fig. 8 shows a methylcellulose cytotoxicity assay of KHYG-1 +/- pretreatment with antibodies against OCI/AML5;

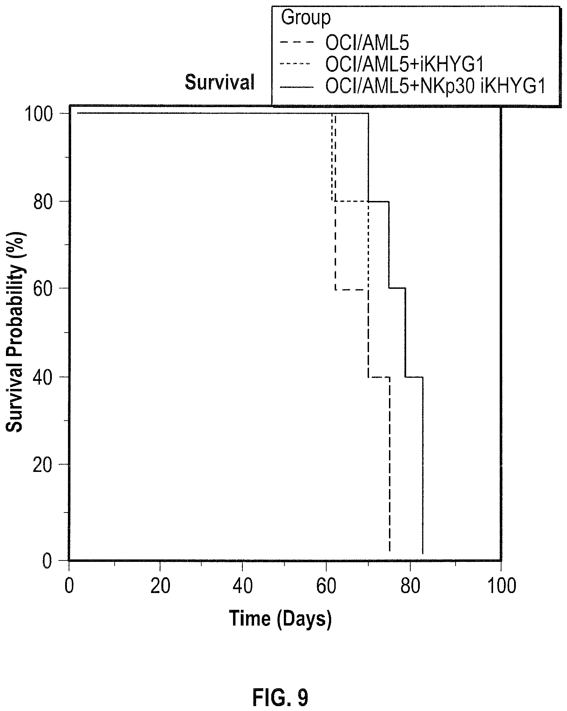

Fig. 9 shows the *in vitro* incubation of OCI/AML5 with iKHYG-1 +/- anti-NKp30 and *in vivo* proliferation in NSG mice;

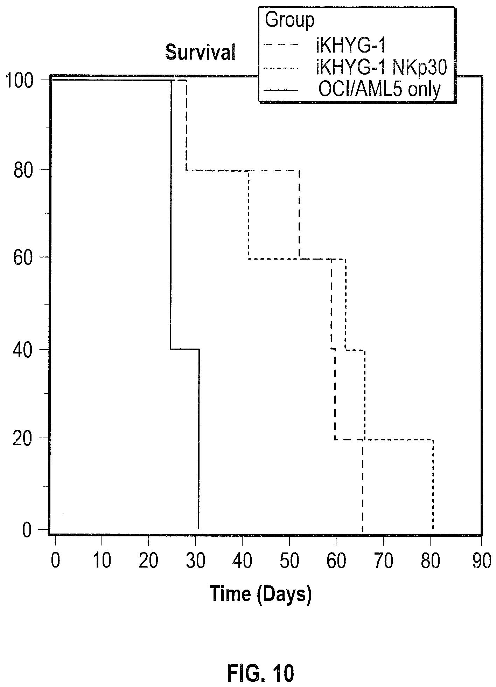

Fig. 10 shows the treatment of OCI/AML5 leukemia in NSG mice with iKHYG-1 +/- NKp30 pretreatment;
Fig. 11 shows the treatment of primary AML xenografted NSG mice with iKHYG-1 +/- NKp30 pretreatment;
Fig. 12 compares the effect of pre-treatment of KHYG-1 with anti-NKp30 antibody and Fab fragments of the antibody on cytotoxicity against leukemia cell lines;
Fig. 13 compares the cytotoxicity of anti-NKp30-coated KHYG-1 cells against unsorted OCI/AML5 cells and CD32^{low} OCI/AML5 cells;
Fig. 14 shows the effect of treating primary NK cells with anti-NKp30 or anti-SLAMF7 on cytotoxicity against the leukemia cell line OCI/AML5;
Fig. 15 shows the effect of treating primary NK cells with lenalidomide and anti-NKp30 or anti-SLAMF7 on cytotoxicity against the leukemia cell line OCI/AML5;

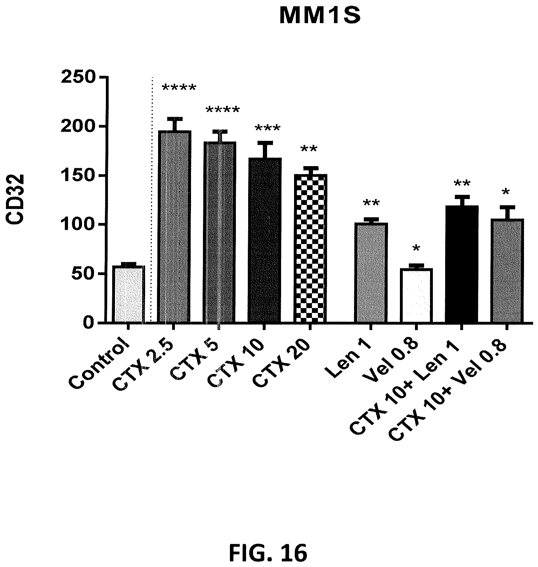

Fig. 16 shows the effect of varying doses of cyclophosphamide on Fc receptor expression on MM1S cells; and

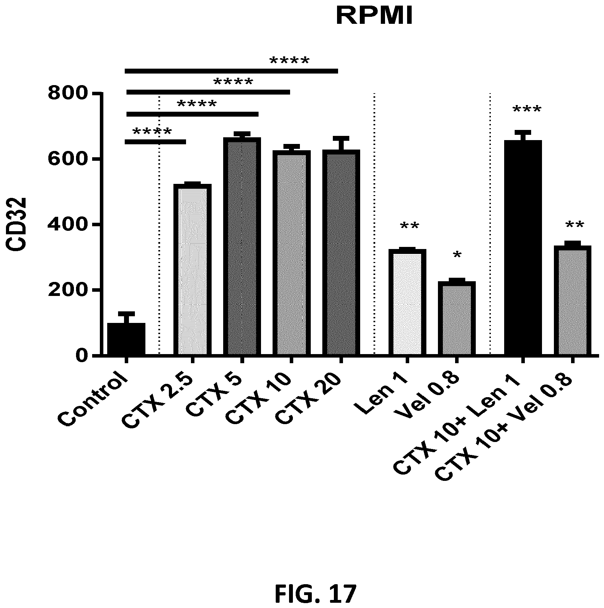

Fig. 17 shows the effect of varying doses of cyclophosphamide on Fc receptor expression on RPMI8226 cells.

In this example of the invention pretreatment of NK cell lines with monoclonal antibodies to activating receptors is demonstrated to cause several-fold enhancement of cytotoxicity against leukemic cell lines and primary AML blasts. This effect was most prominent with anti-NKp30 and anti-NKp44 pretreatment of KHYG-1 against CD32-expressing targets. Further specific work to eliminate other (otherwise credible) explanations showed R-ADCC as the mechanism of enhancement.

We further demonstrated an impact of NKp30 pretreated KHYG-1 in an *in vivo* model.

### Methods

### Cell lines and primary samples

K562 was obtained from the ATCC and maintained in IMDM + 20% FBS and 10% fetal bovine serum (FBS), respectively. KG1 and KG1 a was obtained from the ATCC and maintained in IMDM + 20% FBS and 10% FBS, respectively. OCI/AML 2, 3 and 5 were derived at the Ontario Cancer Institute (OCI). OCI/AML 2 and 3 were cultured in MEM alpha + 10% FBS and OCI/AML5 was cultured in MEM alpha + 10% FBS and 10% 5637 bladder carcinoma condition medium. KHGY-1 was purchased from The Human Science Research Resources Bank (JCRB0156; Tokyo, Japan) and cultured in GM1 (Ex Vivo medium with 450 U/ml and human A/B serum). NK-92 was obtained from Dr. Hans Klingemann and also cultured in Ex Vivo with human A/B serum and 450 U/ml of IL-2 (GM1). KHYG-1 was irradiated (iKHYG-1) with 1000 cGy prior to use in *in vivo* experiments. Five primary AML samples were obtained from the Princess Margaret Hospital Leukemia Tissue Bank as per institutional protocol (5890, 080179, 080078, 080008, 0909).

### Chromium release assay

We utilized a standard chromium release assay as previously described by our group (Williams, Wang et al. 2010) and detailed in the Chapter 2 methods section. Briefly, 1x10⁶ target cells were labelled with 100 µCi of Na₂⁵¹CrO₄ for 2 hours prior to plating 10 000 cells per wells followed by treatment with NK-92 at various concentrations. The amount of ⁵¹Cr present in supernatants was determined using a gamma counter and percent lysis calculated.

### Antibody pretreatment of NK cell effectors

All antibodies used were from Biolegend. For NK pretreatment experiments, antibodies against the following NK receptors were utilized (clone; product #): NKp30 (clone P30-15; 325204), NKp44 (clone P44-8; 325104), NKp46 (clone 9E2; 331904), DNAM-1 (clone DX-11; 316802), NKG2D (clone 1D11; 320810), CD7 (CD7-6B7; 343102). Isotype controls specific to trinitrol phenol + KLH were utilized: MG1-45 (clone MG1-45; 401404) and MG2a-52 (clone MG2a-53, 401502). Briefly, 1.5x10⁶ NK cells (NK-92 or KHYG-1) were treated with 1 ml of AIM-V serum free medium for 1 hour, washed in 10 ml of AIM-V medium and resuspended in 1.5 ml of AIM-V medium (1x10⁶/ml). Concentration of antibodies ranged from 10 µg/ml to 0.01 µg/ml. 0.1 µl (10⁵ cells) of NK cell suspension was added to 10 000 tumour targets also in AIM-V medium in 96 well U bottom plates to yield a 10:1 E:T ratio.

### Flow cytometry

Immunophenotyping of BM was done using an FC500. FACS buffer was made with PBS +2mM EGTA + 2% FBS. For routine flow cytometry of leukemia and esophageal cancer cell lines the following antibodies to Fcγ receptors were utilized: CD16 APC (clone 3G8, 302011), CD32 PE (clone FUN-2, 303205), CD64 FITC (clone 10.1; 305005). Antibody concentrations were utilized at ∼1 µg/ml in a 50 µl reaction volume with 200 000 to 1,000,000 cells.

### High throughput sampling flow cytometry

Commercially validated FITC, PE or APC conjugated antibodies (374) to cell surface markers (BD Pharmingen, eBioscience, Abcam, AbD Serotech, BioLegend, Lifespan Biosciences, Miltenyi, R&D Systems, Beckman-Coulter, and Imgenex) were aliquotted into individual wells of 96-well plates in Hanks Balanced Salt Solution supplemented with 1% bovine serum albumin and 2 mM EDTA (FACS buffer) at a dilution of 1:25 (Supplemental Table 1, 2). NK-92 or KHYG-1 cells (30x10e6) were prepared in 10 ml of PBS, spun down and resuspended in HBSS + 1% BSA, 2mM EDTA and volume adjusted to 1x106/ml. 50 µl of cell suspension (50 000 cells) suspension was added to each well to yield a final antibody dilution of 1:50. Cells were stained for 30 minutes on ice at a concentration of 0.25-1.0x106/mL, washed once with cold FACS buffer, and resuspended in FACS buffer with 0.1 µg/mL DAPI to allow for dead cell exclusion. Flow cytometry was performed using a High Throughput Sampler-equipped Becton-Dickinson LSRII flow cytometer. Plates were placed into an automated flow cytometry plate reader. Data was acquired and analyzed on FlowJo 9. Gating strategy utilized both a FS and SS plot and subsequent DAPI staining to exclude non-viable cells, followed by FSC-H and FSC-W to exclude doublets. Final gate was contoured around viable, unstained cells. Percentage positive cells and mean fluorescence intensity were quantitated for each marker.

### Animals

NOD/SCID gamma^{null} (NSG) mice from The Jackson Laboratory were bred and maintained in the Ontario Cancer Institute animal facility according to protocols approved by the Animal Care Committee. Mice were fed irradiated food and Baytril containing water *ad libitum* during experimental periods. Prior to infusion with AML NSG mice were irradiated with 200 cGy to facilitate engraftment. We developed *ip* and *iv* injection route OCI/AML5 NSG xenograft models utilizing a dose of 2x10⁶ cells. To determine impact of *in vitro* incubation with iKHYG-1 on proliferative capacity of OCI/AML5 the *ip* route of injection was utilized with sacrifice at humane endpoints. Briefly, OCI/AML5 cells were incubated in 15 ml conical tubes with or without iKHYG-1 (+/- 1 µg/ml anti-NKp30 pretreatment x 1 hour) at a 10:1 E:T ratio, spun at 1200 rpm to pellet and incubated for four hours at 37°C. Cell mixtures were then washed and resuspended in PBS and 2x10e6 OCI/AML5 cells with or without 20x10⁶ iKHYG-1 or NKp30 iKHYG-1 cells in 200 µl of PBS were injected *ip* into three cohorts of five NSG mice.

To determine *in vivo* the effect of NK cell line therapy OCI/AML5 or primary AML were injected *iv* on day 0 with and without iKHYG-1 or anti-NKp30 pretreated iKHYG-1 treatment started on day 3 (10x10⁶ x 6 doses; days 3, 5, 7, 10, 12, 14). The primary AML sample (080179) was derived from an M4 leukemia with aggressive engraftment features and passaged through NSG mice prior to use in these experiments.

### Calcein cytotoxicity assay

OCI/AML5 cells were knocked down for CD32 using three siRNA products targeting CD32 a, b and c. Subsequently, OCI/AML5 CD32 knocked down cells were sorted on a cell sorter to acquire the CD32 low fraction, setting acquisition gates to the bottom 10% population. NK cell cytotoxicity against OCI/AML5 and CD32 low OCI/AML5 cells lines was determined using the calcein cytotoxicity assay. Target cells were labeled with 10 µM of calcein-AM for 30 minutes, before 2 washes in serum free RPMI media. Cells were then resuspended at 5x104/ml in AIM-V serum free medium and 100 µl of the cell suspension added to a U bottom 96 well plate. Both NK-92 and CD16+IL-2+NK-92 cells were used as effector cells at a 10:1 Effector:Target (E:T) ratio. After effector cell addition to targets, plates were spun at 500 g for 1 min. Triton X (2%) was used to determine the maximum calcein release. Plates were incubated at 37°C for 2 hours before 75 µl of the supernatant was transferred to a new plate for reading at 480/530 nm.

### Statistics

Survival analysis was done with Kaplan Meier survival curves using the log rank rest with Medcalc software. Comparison of cytotoxicity and engraftment data was done using a two tailed student's t-test performed on Medcalc software. Linear regression analysis was done using Medcalc software and used to generate scatter plots with best fit line, coefficients of determination (R²), F test and degree of significance.

### Results

### NK-92 and KHYG-1 cytotoxicity against leukemia cell lines

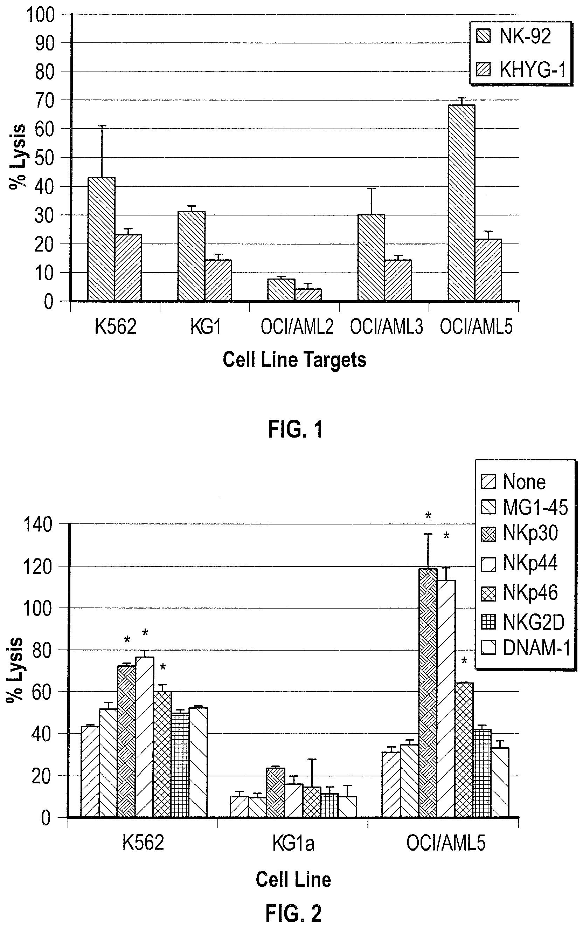

NK-92 and KHYG-1 were tested against a panel of leukemic cell lines (K562, KG1, OCI/AML2, 3 and 5) at a 10:1 E:T ratio using the chromium release assay. Both cell lines demonstrated cytotoxicity against these targets, with NK-92 showing overall better cytotoxicity than KHYG-1 (Fig. 1). OCI/AML5 was particularly sensitive to NK-92 killing with percentage lysis of 68%, exceeding that for K562. OCI/AML2 was relatively resistant to killing by both cell lines with minimal cytotoxicity demonstrated.

NK-92 cytotoxicity against K562 was completely abrogated by calcium chelation at all effector targets ratios, indicating that granule exocytosis was the primary means of cytotoxicity. However, there was a small amount of residual killing of K562 by KHYG-1 particularly at effector:target ratios of 1:1 and 5:1.

### Effect of pretreating NK-92 and KHYG-1 with activating receptor specific antibodies

We attempted to address the role of common activating receptors in NK cell line-mediated recognition of leukemic targets by pretreating NK-92 and KHYG-1 with antibodies specific to NKp30, NKp44, NKp46, DNAM-1, NKG2D, and CD7 (10 µg/ml), prior to co-incubation with the target cells K562, KG1 a and OCI/AML5. An off-target antibody was used as an isotype control. Pretreatment of NK-92 with antibodies to NKp30, NKp44 and NKp46 unexpectedly increased killing of K562 above isotype control [1.3X (p<0.0001), 1.2X (p<0.05), 1.2X (p=0.11)], while anti-NKp30 treatment enhanced killing of KG1 a [+1.8X (p<0.0001)] and OCI/AML5 [1.2X (p<0.01)].

Treatment of KHYG-1 with antibodies to NKp30, NKp44 and NKp46 increased killing of K562 above isotype control [1.4X (p<0.001), 1.5X (p<0.01), 1.2X (p<0.05)], while anti-NKp30 treatment increased killing of KG1 a [2.6X (p<0.01)] and anti-NKp30, anti-NKp44 and anti-NKp46 treatment increased killing of OCI/AML5 [3.4X (p<0.00001), 3.2X (p<0.0001), 1.8X (p<0.0001)] (Fig. 2). Pretreating NK cell lines with antibodies to DNAM-1 and NKG2D had minimal effects on cytotoxicity.

We then attempted a similar experiment with K562 and two primary AML cell lines. NK-92 and KHYG-1 were pre-treated with antibodies specific to NKp30, NKp44, NKp46, DNAM-1, NKG2D and CD7 (10 µg/ml) prior to co-incubation with the leukemic target cells K562, and the primary AML specimens 080078 and 0909. NK-92 cytotoxicity against primary AML samples demonstrated prominent increases above isotype control when pretreated with anti-NKp30 [7.1X (p<0.001) and 3.0X (p<0.0001)].

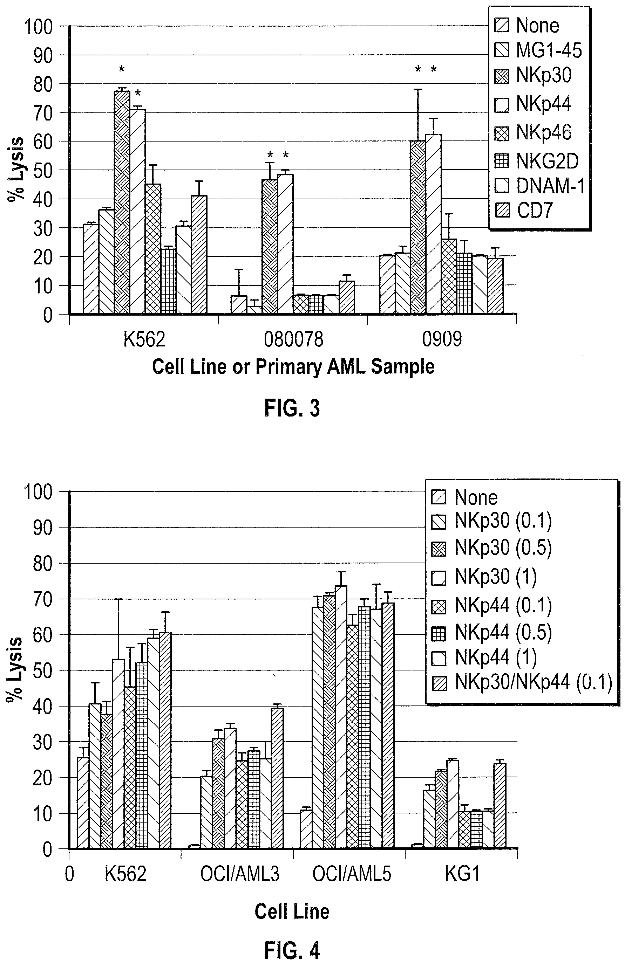

Pretreatment of KHYG-1 with either anti-NKp30 or anti-NKp44 led to dramatic increases of cytotoxicity relative to isotype control against primary AML samples 080078 [16.9X (p<0.0001) and 17.6X (p<0.001)] and 0909 [2.8X (p<0.0001) and 2.9X (p<0.001)] (Fig.3). The dose dependence of anti-NKp30 and anti-NKp44 mediated enhancement of killing was then explored by testing several dose ranges.

In an attempt to determine the linear portion of the dose response curve and approximate the EC50%, pretreatment of NK cell lines was done with a range of doses of anti-NKp30 and anti-NKp44 (1, 5 and 10 µg/ml) against K562, OCI/AML3 and OCI/AML5 and primary AML 080078. A dose response was seen with anti-NKp30 pretreatment of NK-92 against OCI/AML3 and primary AML sample 080078 however the EC50% was less than the lowest dose used (1 µg/ml) (data not shown). KHYG-1 had a similar degree of enhancement seen at all dose ranges of both anti-NKp30 and anti-NKp44 antibody pretreatments (data not shown).

A lower dose range was then selected utilizing NK cell lines pretreated with isotype control, anti-NKp30 and anti-NKp44 at 0.1, 0.5 and 1 µg/ml. Isotype-control pretreated NK-92 and KHYG-1 had minimal effects on cytotoxicity against K562, OCI/AML3, OCI/AML5 and KG1, with no dose response. Anti-NKp30 pretreatment enhanced NK-92 cytotoxicity against OCI/AML3 (EC50% ∼0.1 µg/ml) and KG1 (EC50%<0.1 µg/ml) only. Combined anti-NKp30 and anti-NKp44 pretreatment (0.1 µg/ml) of NK-92 did not have additive or synergistic effects on cytotoxicity against any targets.

Anti-NKp30 pretreatment enhanced KHYG-1 cytotoxicity against all targets with a dose response seen for K562 (EC50% ∼0.1 µg/ml), OCI/AML3 (EC50% <0.1 µg/ml), and KG1 (EC50% <0.1 µg/ml), while OCI/AML5 showed a plateau from the lowest dose (EC50% <0.1 µg/ml). Anti-NKp44 pretreatment enhanced KHYG-1 cytotoxicity against all targets, with a dose response seen for K562 only (EC50% ∼0.1 µg/ml), while OCI/AML3, KG1 and OCI/AML5 showed a plateau from the lowest dose (EC50% <0.1 µg/ml) (Fig. 4).

Combined anti-NKp30 and anti-NKp44 pretreatment (0.1 µg/ml) of KHYG-1 demonstrated greater enhancement on cytotoxicity than each antibody alone against K562, OCI/AML3 and KG1, but not OCI/AML5.

Not only did the combined dosing of 0.1 µg/ml anti-NKp30 and anti-NKp40 exceed the effect of each antibody alone at this dose level, it also matched or exceeded the effect of a 10-fold higher dose (1 µg/ml) of each antibody alone for K562, OCI/AML3 and KG1, demonstrating true synergy.

The % lysis values for untreated, isotype control, anti-NKp30, anti-NKp40 and combined anti-NKp30 and anti-NKp44 groups (0.1 µg/ml) and anti-NKp30 and anti-NKp44 (1 µg/ml) for the cell line targets treated with KHYG-1 are tabulated for comparison (Table 1).

**Table 1: Comparison of antibody pretreatment effects on KHYG-1 cytotoxicity and synergy assessment at 0.1 µg/ml**

| **Cell line** | **% Lysis (mean +/- SD)** | | | | | | |
|---|---|---|---|---|---|---|---|
| | **Pretreatment** | | | | | | |
| | **0.1 µg/ml** | | | | | **1.0 µg/ml** | |
| | **None** | **MG2a-53** | **Anti-NKp30** | **Anti-NKp44** | **Anti-NKp44/ NKp30** | **Anti-NKp30** | **Anti-NKp44** |
| K562 | 26 +/- 3.2 | 37 +/- 2.9 | 41 +/-6.0 | 46 +/-11.1 | ***61 +/- 5.9** | 54 +/-16.7 | 59 +/-2.6 |
| OCI/AML3 | 0 +/- 0.8 | 9 +/-7.8 | 20 +/- 2.0 | 25 +/- 1.5 | ***39 +/-1.7** | 34 +/-1.3 | 26 +/-4.8 |
| OCI/AML5 | 11 +/- 0.5 | 19 +/- 3.1 | 68 +/- 2.7 | 62 +/- 3.5 | 69 +/- 3.8 | 74 +/-42 | 67 +/- 7.0 |
| KG1 | 1 +/- 0.4 | 9 +/- 1.7 | 16 +/- 1.7 | 10 +/- 2.2 | ***24 +/- 1.1** | 25 +/-0.5 | 11 +/- 0.7 |

| | | | | | | | |
|---|---|---|---|---|---|---|---|
| *Combined anti-NKp30 and anti-NKp44 regimens that yielded statistically significant (p<0.05) increases above antibodies alone in separate comparisons are in bold font. | | | | | | | |

While dose responses could be seen in the range of 0.1 to 1 µg/ml in many cases, the lowest dose exceeded the EC50%. Therefore, an additional experiment testing a dose range one log lower was conducted (0.01, 0.1 and 1 µg/ml). There was minimal effect of the isotype control (MG2a-53) antibody in this range, with no dose response seen (data not shown). Pretreatment of NK-92 with 0.01, 0.1 and 1 µg/ml of anti-NKp30 enhanced cytotoxicity of OCI/AML3 only (ED50% 0.01 to 0.1 µg/ml) and there was no effect of anti-NKp44 pretreatment.

Anti-NKp30 and anti-NKp44 pretreatment enhanced KHYG-1 cytotoxicity against all targets with a dose response seen for K562, OCI/AML3 and OCI/AML5 (EC50% 0.01-0.1 µg/ml).

Combined anti-NKp30 and anti-NKp44 pretreatment of KHYG-1 (0.01 µg/ml) demonstrated synergistic effects on cytotoxicity against OCI/AML3 only at this dose level.

The combined dosing of 0.01 µg/ml anti-NKp30 and anti-NKp40 exceeded the effect of each antibody alone (2X) and the absolute cytotoxicity was comparable to a 10-fold higher dose of each antibody (0.1 µg/ml). The % lysis values for untreated, isotype control, anti-NKp30, anti-NKp40 and combined anti-NKp30 and anti-NKp44 groups (0.01 µg/ml) and anti-NKp30 and anti-NKp44 (1 µg/ml) for the cell line targets treated with KHYG-1 and its isotype controls are tabulated for comparison (Table 2).

**Table 2: Comparison of antibody pretreatment effects on KHYG-1 cytotoxicity and synergy assessment at 0.01 µg/ml**

| **Cell line** | **% Lysis (mean +/- SD)** | | | | | | |
|---|---|---|---|---|---|---|---|
| | **Pretreatment** | | | | | | |
| | **0.01 µg/ml** | | | | | **0.1 µg/ml** | |
| | **None** | **MG2a-53** | **Anti-NKp30** | **Anti-NKp44** | **Anti-NK44/ NKp30** | **Anti-NKp30** | **Anti-NKp44** |
| K562 | 21 +/- 1.6 | 29 +/- 2.4 | 28 +/- 6.0 | 26 +/-2.5 | 32 +/- 2.0 | 35 +/- 0.5 | 41 +/- 3.4 |
| OCI/AML3 | 4 +/- 0.1 | 7 +/- 1.8 | 7 +/-0.8 | 7 +/- 0.6 | **13*** +/- 3.8 | 13 +/- 0.5 | 19 +/- 3.4 |
| OCI/AML5 | 12 +/- 0.8 | 16 +/- 5.0 | 38 +/- 1.9 | 30 +/- 3.8 | 36 +/- 7.6 | 65 +/- 1.5 | 63 +/- 5.4 |
| KG1 | 9 +/- 7.4 | 9 +/- 1.2 | 6 +/- 1.0 | 9 +/- 4.7 | 11 +/- 1.0 | 9 +/- 1.1 | 15 +/- 4.5 |

| | | | | | | | |
|---|---|---|---|---|---|---|---|
| Combined anti-NKp30 and anti-NKp44 regimens that yielded statistically significant (p<0.05) increases above antibodies alone in separate comparisons are in bold font. | | | | | | | |

To determine if anti-NKp30 and anti-NKp44 pretreatment of NK cell lines could enhance cytotoxicity against a solid tumour, we performed the same experiment with esophageal cancer cell lines (FLO-1, OE-33, SKGT-4, KYAE-1). However, pretreatment of NK-92 and KHYG-1 with 0.1 µg/ml of anti-NKp30 and anti-NKp44 mAb did not enhance cytotoxicity against four esophageal cancer cells lines relative to the isotype control. This suggested the presence of a unique cell surface marker present on leukemia cells, but not esophageal cancer cells, responsible for mediating the enhancing effect of antibody-pretreated NK cell lines.

### Relationship of Fcγ receptor expression and enhancement of cytotoxicity

We conducted HTS flow cytometry on two representative leukemic cell line targets (OCI/AML3 and OCI/AML5) to assess for potential cell surface markers that might be involved in enhancing the cytotoxicity of anti-NKp30 or anti-NKp44 coated NK cell lines. We noted a high degree of CD32 (FcγRII) expression on both cell lines. Subsequently, we conducted routine flow cytometry on all leukemic and esophageal cancer cell lines for expression of Fcγ receptors (CD16, CD32 and CD64). The leukemia cell lines showed relatively high expression of Fcγ receptor II (CD32), but very low expression of Fcγ receptor I (CD64) or Fcγ receptor III (CD16) on leukemia lines K562, KG1, KG1a, OCI/AML3, and OCI/AML5.

The histogram shape for K562 suggested the presence of both intermediate and high CD32 expressing subpopulations. KG1 a appeared to have a dual population of negative and low CD32 expressing subpopulations. OCI/AML3 had a clear dual peak representing two high CD32 expressing subpopulations. There were clear single populations of CD32 expressing cells in KG1 (moderate) and OCI/AML5 (high). There was no significant expression of Fcγ receptors on esophageal cancer lines (OE-33, FLO-1, KYAE-1 and SKGT-4).

The percent positivity of each leukemic and esophageal cancer cell line was determined from flow cytometry plots. Data from prior cytotoxicity assays measuring the enhancement of cytotoxicity of NK-92 and KHYG-1 when pretreated with 10 µg/ml of either anti-NKp30 or anti-NKp44 antibody were compared to isotype controls and delta cytotoxicity calculated. The delta cytotoxicity relative to isotype control was correlated with the degree of CD32 expression using regression analysis to create best fit lines, calculate co-efficient of determination (R²) and statistical significance. Regression analysis of the relationship of delta cytotoxicity of antibody-pretreated NK-92 with CD32 expression of targets did not reveal a correlation for anti-NKp30 (R²=0.13; p=0.34) and anti-NKp44 (R²=0.22; p=0.20) pretreatments (A and B).

However, regression analysis of the relationship of delta cytotoxicity of antibody pretreated KHYG-1 with CD32 expression of targets revealed a correlation for both anti-NKp30 (R²=0.71; p<0.01) and anti-NKp44 pretreatments (R²=0.64; p<0.01) (C and D).

### Effect of anti-NKp30 pretreatment on NK cell line cytotoxicity against clonogenic OCI/AML5

To determine the effect of anti-NKp30 and anti-NKp44 pretreated NK cell lines against clonogenic leukemic cells, we utilized our previously established clonogenic cytotoxicity assay utilizing OCI/AML5 as the target. Comparison of killing was made with untreated, isotype control (MG1-45) and anti-CD7 pretreated NK cell lines. CD7 is highly expressed on NK-92 and KHYG-1, with no confirmed activating capacity in these cell lines. Therefore, anti-CD7 antibody pretreatment was chosen as an additional control. There was no difference between isotype control and CD7-pretreated NK-92. Pretreatment of NK-92 with 0.1 µg/ml anti-NKp30 had only a slight impact (+3.7%; p<0.05) on OCI/AML5 clonogenic capacity relative to baseline and isotype control.

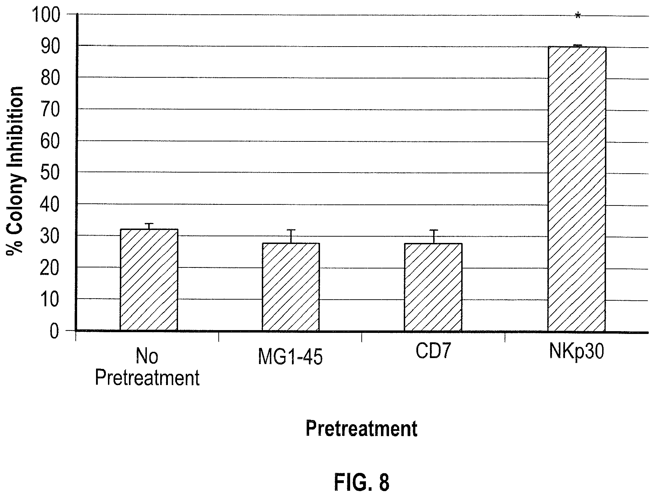

However, pretreatment of KHYG-1 with anti-NKp30 enhanced % colony inhibition 3-fold (+63.1%; p<0.0001) relative to baseline and isotype control (Fig. 8). While the baseline % colony inhibition of NK-92 (61.9 %) was greater than KHYG-1 (32.0%), anti-NKp30 pretreated KHYG-1 (90.7%) had the greater inhibition relative to NK-92 (+28.8%; p<0.0001).

### In vitro effect of anti-NKp30 pretreated iKHYG-1 against OCI/AML5 capacity for leukemic progression in an NSG xenograft model

We tested the *in vitro* cytotoxic effect of KHYG-1 on *in vivo* progression of leukemia in an OCI/AML5 xenograft model. KHYG-1 proliferation was prevented by irradiation with 1000 cGy prior to use. OCI/AML5 cells were co-incubated with irradiated KHYG-1 (iKHYG-1) +/-1 µg/ml anti-NKp30 pretreatment for one hour prior to a 4 hour co-incubation at a 10:1 E:T with OCI/AML5 cells. Subsequently, cell mixtures were injected *ip* into three cohorts of NSG mice with survival as an endpoint. Individual NSG mice were injected with 2x10⁶ OCI/AML5 cells +/- 20x10⁶ viable effector cells (iKHYG-1 or anti-NKp30 pretreated iKHYG-1). At 9 weeks, control mice developed progressive malignant ascites with minimal splenomegaly and imbedded vascular tumours in the omentum.

Co-incubation with iKHYG-1 did not improve survival (p=0.92). However, anti-NKp30 pretreated iKHYG-1 improved survival compared to the no therapy (p<0.05) or iKHYG-1 (p<0.05) cohorts.

### Effect of anti-NKp30 pretreatment of iKHYG-1 on therapeutic efficacy for OCI/AML5 or primary AML xenografted mice

We evaluated OCI/AML5 engraftment potential in NSG mice by infusing 5x10⁶ OCI/AML5 cells via tail vein and measured bone marrow engraftment at two weeks. Bone marrow engraftment of OCI/AML5 was detected by measuring human CD33 expression in bone marrow samples and revealed relatively rapid, but variable bone marrow engraftment (13.0, 52.3, 29.9, 63.5%). In a subsequent survival endpoint experiment, 2x10⁶ OCI/AML5 cells were injected *iv* into cohorts of five mice. OCI/AML5-xenografted mice were then treated without and with iKHYG-1 or anti-NKp30 pretreated iKHYG-1 (10x10⁶ x 6 doses *ip*). There was significant improvement in survival of mice treated with either iKHYG-1 (+35 days median survival; p<0.05) or anti-NKp30 pretreated iKHYG-1 (+37 day median survival p<0.05) above control.

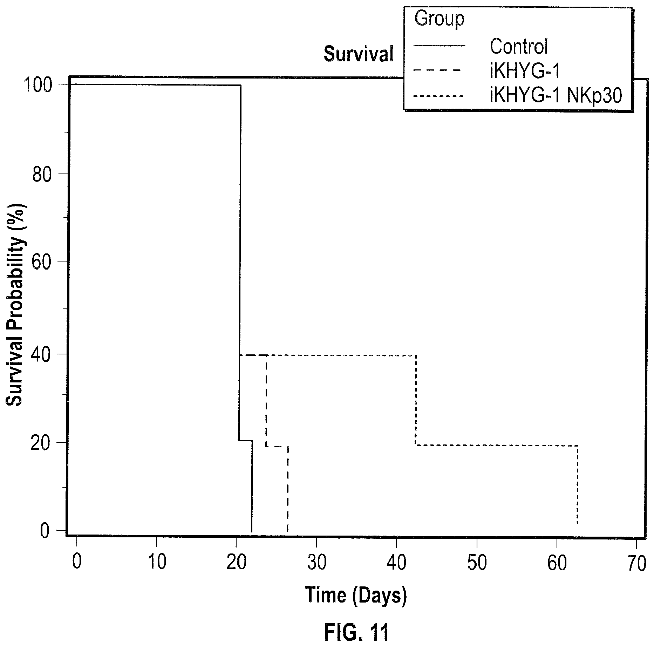

We utilized a primary AML sample (080179) known to engraft and cause leukemia in NSG mice as a model to test the efficacy of irradiated NK-92 and KHYG-1 pretreated with and without anti-NKp30 (1 µg/ml) prior to injection into NSG mice inoculated with primary AML. iKHYG-1 and iNK-92 did not prolong survival in the primary AML model, although iKHYG-1 pretreated with anti-NKp30 showed some longer term survivors (3-4 weeks above control median) with a trend toward significance (p=0.20) versus iKHYG-1 alone (Fig. 11).

### Elucidation of cytotoxicity mechanism

Work thus far demonstrated that pretreatment of KHYG-1 with anti-NKp30 antibodies lead to greatly enhanced cytotoxicity against several cell lines and primary AML targets. However, there are many potential mechanisms that could explain this experimental data, leading to doubt as to the utility of the observations and as to application thereof in therapy.

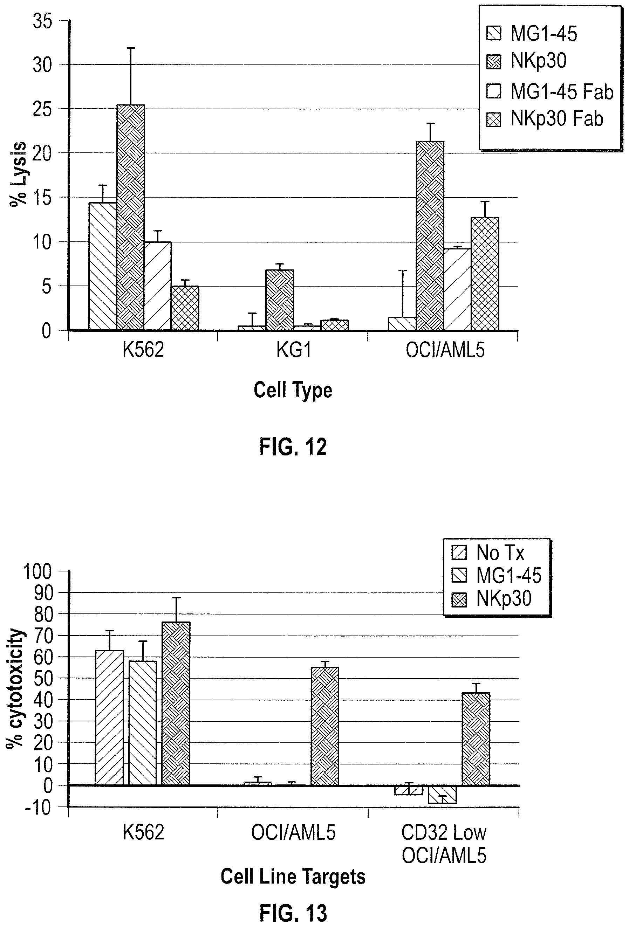

We devised further experimental work to divine the actual mechanism. Specifically, Fab fragments of the anti-NKp30 (P30-15) clone and isotype control (MG1-45) were generated. KHYG-1 was precoated with 0.1 µg/ml of anti-NKp30 and isotype control whole antibody or Fab fragments for 1 hour and then washed in AIM-V medium. Precoated KHYG-1 was then used in a four hour chromium release assay against K562, KG1 and OCI/AML5 targets (Fig. 12). Only anti-NKp30 antibody was able to significantly enhance cytotoxicity against the three cell lines, while the anti-NKp30 antibody fragment either blocked cytotoxicity (K562), had no impact on cytotoxicity (KG1) or had minimal effects above isotype control Fab (OCI/AML5).

We further devised an experiment in which Fc receptor expression on the target cells was reduced. Specifically, to determine target CD32 dependence of the antibody-mediated enhancement of KHYG-1 cytotoxicity, as proof R-ADCC is the mechanism responsible, we attempted to use siRNA knockdown of the three gene isoforms of CD32 (a, b and c). While we were unable to achieve complete CD32 knockdown in the OCI/AML5 cell line, we were able to use cell sorting to acquire a CD32 low OCI/AML5 population for subsequent testing in a calcein cytotoxicity assay.

Unsorted OCI/AML5 targets were resistant to KHYG-1 cells, unless they were pretreated with 0.1 ug/ml of anti-NKp30 antibody, leading to a high degree of cytotoxicity at a 10:1 effector:target ratio, near equivalent to the NK sensitive K562 cell line. While CD32 low OCI/AML5 were sensitive to anti-NKp30 pretreated KHYG-1 cells, the degree of enhancement was 12% less (p=0.02) than for unsorted OCI/AML5 (Fig. 13).

In the context of this culture environment, in which the NK cells are localized with the target cancer cells, this is a highly significant effect and demonstrates that the enhancement in cytotoxicity is dependent on Fc gamma receptor II (CD32) expression on the target, further supporting R-ADCC as the mechanism of antibody enhanced cytotoxicity of KHYG-1 cells.

Therefore, the mechanism of enhancement was determined to be R-ADCC and not co-activation, co-stimulation or any other effect. Progress in developing therapies based on the totality of the work herein was thus made possible.

### Fc receptor assay protocol

Fc receptor expression on cancers can be identified and quantitated using flow cytometry, using methods known to the person skilled in the art. A biopsy of the cancerous tissue is acquired from the patient and cultured in the appropriate growth medium. Cancer cells are isolated from the tissue and analysed as follows.

50µL of cell suspension (containing approximately 2x10⁶ cells) in FACS buffer is added to each FACS tube. The fluorochrome-conjugated mAb to the Fc receptor or cancer marker of interest is then added at the manufacturer's recommended volume and the total volume made up to 100µL with FACS buffer. This mixture is vortexed and left to incubate at room temperature in the dark for approximately 15 minutes. Following incubation, the cells must be washed by adding 3mL FACS buffer to each tube and pelleting the cells via centrifugation. This wash step must be repeated twice.

The cells are then re-suspended in an appropriate volume of FACS buffer for data acquisition via flow cytometry.

Expression of cancer-specific markers and Fc receptors on the cell surface is analysed. Cells found to be +/+ for the marker and Fc receptor are treatable using the invention. Therapeutic antibody doses (required for R-ADCC) may be calculated based on the Fc receptor expression level of a given cancer, i.e. more antibody might be required for cancer expressing lower levels of Fc receptors and vice versa.

### Therapeutic protocol for use with autologous NK cells

The following protocol was developed for cancer treatment using autologous NK cells:
Following diagnosis of a patient with a cancer suitably treated with the invention (see Fc receptor assay above), a peripheral blood sample is obtained. Peripheral blood mono-nucleated cells (PBMCs) can be harvested using a Ficoll separation technique and then negative magnetic separation (MACS) can be used to isolate NK cells from the PBMCs.

Once in a suitable culture medium, e.g. RPMI 1640 + 10% FBS + 2mM L-Glutamine + 1mM PenStrep, the NK cells are briefly expanded before being treated with activating agent(s) such as lenalidomide and/or IL-2.

The NK cells are then re-suspended in a suitable carrier (e.g. saline) for intravenous injection back into the bloodstream of the patient and/or intratumoural injection. The therapeutic (R-ADCC) antibody is injected prior to, simultaneously with or subsequently to the NK cells.

Alternatively, the therapeutic (R-ADCC) antibody can be used to treat the NK cells in culture. This results in a requirement for only one injection, as the antibody is attached to the cells upon administration. Furthermore, it limits the extent to which non-activated endogenous NK cells are targeted by the antibody.

### Therapeutic protocol for use with endogenous NK cells

The following protocol was developed for cancer treatment using endogenous NK cells:
Following diagnosis of a patient with a cancer suitably treated with the invention (see Fc receptor assay above), a suspension of activating agent(s), such as lenalidomide and/or IL-2, in a suitable carrier (e.g. saline) is injected into the bloodstream of the patient and/or directly into the tumour. The therapeutic (R-ADCC) antibody can be injected prior to, simultaneously with or subsequently to the activating agent(s).

Alternatively, the activating agent(s) can be mixed with the therapeutic (R-ADCC) antibody in a single suspension and administered to the patient in unison.

### Effect of Anti-NKp30 Antibody and Anti-SLAMF7 Antibody on NK-92 Induced Target Cell Death

Anti-SLAMF7 (R&D Prod# MAB1906, clone 235614) and anti-NKp30 (clone P30-15; 325204) were used separately to treat NK-92 cells co-cultured with target cells.

A final concentration of 10µg/mL anti-SLAMF7 or 10µg/mL anti-NKp30 was used, with a final concentration of 1% DMSO as the vehicle control. The effector:target (E:T) ratios used for NK-92 effector cells vs. K562 target cells or OCI/AML5 target cells was 10:1. The co-cultures were treated for 4 hours prior to measuring the % lysis via chromium release assay, as discussed above.

Treatment of NK-92 cells with either the anti-SLAMF7 antibody or the anti-NKp30 antibody resulted in a significant increase in the cytotoxicity of NK-92 cells against K562 cells.

However, only treatment with the anti-NKp30 antibody resulted in a significant increase in the cytotoxicity of NK-92 cells against OCI.AML5 cells.

These data clearly indicate an R-ADCC effect using antibodies specific for two different NK cell receptors, not otherwise expressed on the target cell and thus ruling out any ADCC effect.

### Effect of Anti-NKp30 Antibody and Anti-SLAMF7 Antibody on Primary NK Cell Induced Target Cell Death

Anti-SLAMF7 (R&D Prod# MAB1906, clone 235614) and anti-NKp30 (clone P30-15; 325204) were used separately to treat primary NK cells (isolated from healthy human donors the same day) co-cultured with target cells.

A final concentration of 10µg/mL anti-SLAMF7 or 10µg/mL anti-NKp30 was used, with a final concentration of 1% DMSO as the vehicle control. The effector:target (E:T) ratios used for primary NK effector cells vs. OCI/AML5 target cells was 10:1. The co-cultures were treated for 4 hours prior to measuring the % lysis via chromium release assay, as discussed above.

Treatment of primary NK cells with either the anti-SLAMF7 antibody or the anti-NKp30 antibody resulted in a significant increase in the cytotoxicity of primary NK cells against OCI.AML5 cells.

These data clearly indicate an R-ADCC effect using antibodies specific for two different NK cell receptors, not otherwise expressed on the target cell and thus ruling out any ADCC effect. Furthermore, in contrast to NK-92 effector cells, primary NK effector cells exhibited increased cytotoxicity against the OCI.AML5 cell line when treated with the anti-SLAMF7 antibody.

### Effect of Lenalidomide and Anti-SLAMF7 Antibody on KHYG-1 Induced Target Cell Death

Lenalidomide (BOC Sciences CAS 191732-72-6, Batch No. B16YC05152) and anti-SLAMF7 (R&D Prod# MAB1906, clone 235614) were used alone and in combination to treat KHYG-1 cells co-cultured with target cells.

A final concentration of 10µM lenalidomide and 10µg/mL anti-SLAMF7 was used, with a final concentration of 1% DMSO as the vehicle control. The effector:target (E:T) ratios used for KHYG-1 effector cells vs. K562 target cells or OCI/AML5 target cells was 10:1. The co-cultures were treated for 4 hours prior to measuring the % lysis via chromium release assay, as discussed above.

Lenalidomide treatment alone was found to increase the cytotoxicity of KHYG-1 cells against both K562 cells and OCI/AML5 cells.

When lenalidomide was added in addition to anti-SLAMF7, no further increase in OCI/AML5 cell death was observed compared to anti-SLAMF7 treatment alone. However, lenalidomide in addition to anti-SLAMF7 was able to increase K562 cell death when compared to anti-SLAMF7 treatment alone.

These data are preliminary and no statistical analysis of their significance has been carried out.

Nevertheless, the data clearly indicate an ability of lenalidomide to increase NK cell cytotoxicity during R-ADCC, even in the absence of T cells. This therefore indicates that lenalidomide activates NK cells directly, as well as indirectly via induction of IL-2 secretion from T cells, and thus provides further support for the *in vivo* use of activating agents in combination with antibodies capable of R-ADCC.

### Effect of Lenalidomide and Various R-ADCC Antibodies on Primary NK Cell Induced Target Cell Death

Lenalidomide (BOC Sciences CAS 191732-72-6, Batch No. B16YC05152) and either anti-NKp30 (clone P30-15; 325204) or anti-SLAMF7 (R&D Prod# MAB1906, clone 235614) antibodies were used to treat primary NK cells co-cultured with target cells. The antibodies were used individually alone or in combination with lenalidomide, in order to test whether lenalidomide could enhance the R-ADCC effect using primary NK cells at a physiological effector:target (E:T) ratio.

A final concentration of 10µM lenalidomide and 10µg/mL anti-NKp30 or anti-SLAMF7 was used, with a final concentration of 1% DMSO as the vehicle control. The effector:target (E:T) ratios used for primary NK effector cells vs. OCI/AML5 target cells was 1:1. The co-cultures were treated for 4 hours prior to measuring the % lysis via chromium release assay, as discussed above.

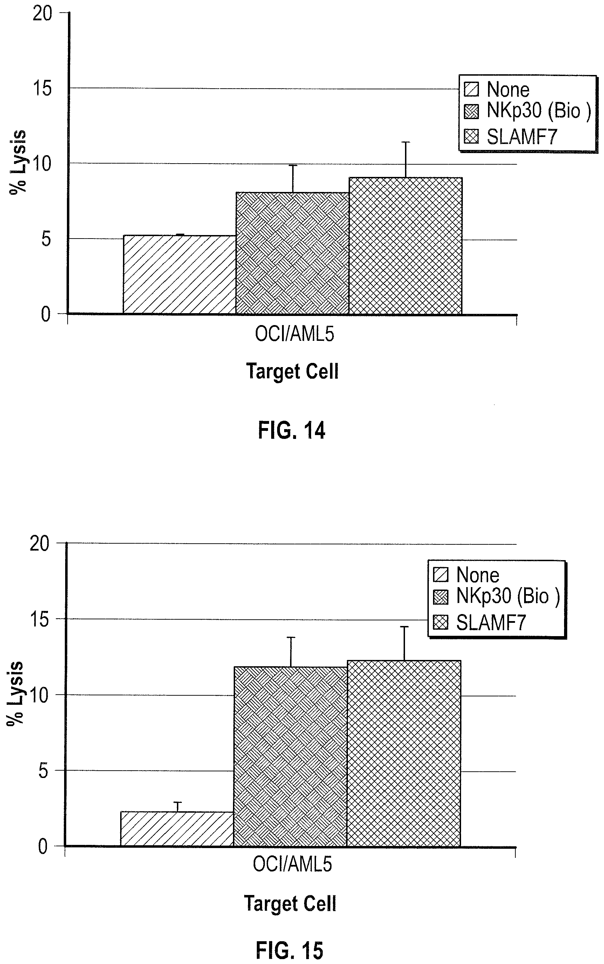

The data showed that even with a physiological E:T ratio of 1:1, both anti-NKp30 and anti-SLAMF7 were able to significantly increase the cytotoxicity of primary NK cells against OCI.AML5 cells (see Fig. 14). The anti-NKp30 and anti-SLAMF7 antibodies resulted in a respective 3% and 4% increase in target cell lysis, compared to primary NK cells alone.

This R-ADCC effect was significantly enhanced by the addition of lenalidomide (see Fig. 15). The anti-NKp30 and anti-SLAMF7 antibodies in combination with lenalidomide resulted in a 10% increase in target cell lysis, compared to primary NK cells alone.

These data provide further evidence that lenalidomide acts directly on NK cells to enhance their cytotoxicity against cancer cells, and that this effect is apparent even in the presence of R-ADCC. Furthermore, the dual effect of R-ADCC and small molecule-induced NK cell activation is effective using primary NK cells, even at E:T ratios as low as 1:1.

### Upregulation of Fc Receptor Expression via All-Trans Retinoic Acid Treatment

Cancer cells that express Fc receptors, in particular CD32, may at times express these receptors at level too low to make use of treatment according to the invention. In such circumstances, and when an enhanced effect of the treatment is desired, it is possible to upregulate expression of Fc receptors on the cancer in the patient.

Upregulation of Fc receptor expression on leukemia cells can be achieved using all-trans retinoic acid (ATRA). ATRA is an FDA-approved chemotherapeutic agent and has been shown to increase expression of CD32 by 30-fold (Barber et al, 2008). ATRA is to be administered to the patient at a dose of 45mg/m² daily and in two divided doses for a duration not exceeding 90 days. Dosing may vary, however, depending on the baseline Fc receptor expression of a given cancer.

Upregulation of Fc receptors on the cancer can also be used to decrease the therapeutic antibody dose (required for R-ADCC) which, in some cases, may be calculated based on the Fc receptor expression level of the cancer.

### Upregulation of Fc Receptor Expression via Cyclophosphamide Treatment

MM1S and RPMI8226 cells were plated in RPMI1640 medium supplemented with 10% FBS and 1% pen/strep at a density of 8 x 10⁵/ml and 5 x 10⁵/ml, respectively, in flat-bottom 6 well plates (2ml/well).

Cells were treated 48hrs after plating when they reached 70% confluency. The cells were exposed to increasing doses (0, 2.5, 5, 10 and 20 micromolar) of the active metabolite of cyclophosphamide, 4-hydroxy cyclophosphamide, for 24 hours. It was known from previous experiments that ≤ 10 micromolar 4-hydroxy cyclophosphamide does not induce significant cell death.

Cells were also exposed to low doses of lenalidomide (1µM) and bortezomib (0.8nM), both alone and in combination with 10 micromolar 4-hydroxy cyclophosphamide.

Cells were washed three times with D-PBS (GibcoTM) and re-plated with fresh RPMI1640 medium supplemented with 10% FBS and 1% pen/strep for 24hours.

The expression of the Fc receptor CD32 on the cells with and without treatment was detected using an anti-human monoclonal antibody to anti-CD32-PE (clone FLI8.26) by flow cytometry.

As shown in Fig.s 16 and 17, respectively, the expression of CD32 on MM1S and RPMI8226 cells was significantly increased by 4-hydroxy cyclophosphamide treatment at all doses used (MFI 62-158 in MM1S, 155-697 in RPMI1640), with the most prominent effect seen with low doses (< 5 micromolar, p < 0.0001).

These data demonstrate a use for cyclophosphamide therapy, in order to up-regulate Fc receptor expression on cancers, and hence be used in combination with R-ADCC therapy.

### Discussion

Here, we have conducted all experiments with KHYG-1 using a clinical grade medium (GM1) that lacks fetal bovine serum and may be suitable for future clinical application. KHYG-1 cytotoxicity against primary AML has not been previously reported. We noted that KHYG-1, without antibody, was less effective than NK-92 at killing both leukemia cell lines and primary AML samples.

We sought to determine the effect of pretreating NK-92 and KHYG-1 with antibodies against a panel of activating receptors commonly associated with NK cell cytotoxicity (natural cytotoxicity receptors, NKG2D and DNAM-1). While we anticipated potential blocking of cytotoxicity at 10 µg/ml against a panel of leukemia cell line targets, we observed stimulation of cytotoxicity from some of the antibodies and no blocking of cytotoxicity. Treatment of NK-92 with antibodies to NKp30, NKp44 and NKp46 increased killing of K562 by approximately 10%, while only anti-NKp30 treatment enhanced killing of KG1a and OCI/AML5. Further, anti-NKp30, but not anti-NKp44 pretreated NK-92 had a large degree of enhancement in killing of primary AML.

HTS flow cytometry demonstrated higher expression of NKp30, NKp44 and NKp46 on KHYG-1 compared with NK-92. Treatment of KHYG-1 with antibodies to all the natural cytotoxicity receptors increased killing of K562 and OCI/AML5 to a greater degree than NK-92. KHYG-1 cytotoxicity against primary AML was enhanced to a greater degree than NK-92 with pretreatment with anti-NKp30 or anti-NKp44. Pretreatment of NK cell lines with antibodies against DNAM-1, NKG2D (both commonly involved in NK cell recognition) induced small statistically significant inhibitory effects against target K562 in some experiments, indicating a possible role for these molecules in recognition. However, anti-DNAM-1 and anti-NKG2D were not able to facilitate reverse ADCC despite high expression of DNAM-1 and NKG2D on both cell lines.

We sought to determine the dose response curve of antibody-mediated cytotoxic enhancement, which was most prominent with KHYG-1 against OCI/AML5. Increases in KHYG-1 cytotoxicity against OCI/AML5 was seen as low as 0.01 µg/ml, which is near the EC50 of the stimulatory effect. A plateau in enhancement started to occur at 0.1 µg/ml with slight increase in efficacy at 1 µg/ml. NK-92 had lesser enhancement than KHYG-1 with this approach, but effects could be observed in the 0.01 µg/ml dose range particularly against OCI/AML3, which is somewhat resistant to NK-92 cytotoxicity.

The antibodies we used were reported as blocking antibodies, based on studies of endogenous NK cell cytotoxicity (Markel, Seidman et al. 2009) - inconsistent with the results observed. Fab fragments of the anti-NKp30 (P30-15) clone and isotype control (MG1-45) were generated. KHYG-1 was precoated with anti-NKp30 and isotype control whole antibody or Fab fragments of each, before co-incubating with K562, KG1 and OCI/AML5 targets. Only anti-NKp30 antibody was able to significantly enhance cytotoxicity against the three cell lines.

Moreover, a population of OCI/AML5 target cells were selected for low expression of CD32, before being co-cultured with KHYG-1 cells, pre-coated with anti-NKp30 antibody. The ability of the anti-NKp30--coated KHYG-1 cells to kill the CD32^{low} OCI/AML5 target cells was significantly reduced, when compared to a group of unsorted OCI/AML5 cells expressing higher levels of CD32.

These unexpected but advantageous findings show that the increased cytotoxicity was via reverse ADCC.

The R-ADCC mechanism responsible for the increased cytotoxicity observed was further confirmed via the finding that four esophageal cell lines, known to express no or low levels of Fc receptors, were not responsive to treatment with KHYG-1 cells, pre-coated with either anti-NKp30 antibody or anti-NKp44 antibody.

Hence, the invention uses R-ADCC as a means of enhancing NK cell line cytotoxicity - in examples against leukemic cell lines and, more importantly, primary AML cells, but also of wider application.

We then sought to determine if the enhancing effect of pretreating NK cell lines with anti-NKp30 and anti-NKp44 held against clonogenic cells using our established methylcellulose cytotoxicity assay. We noted that NK-92 was relatively effective at inhibiting OCI/AML5 colony formation, but this could not be enhanced by pretreatment with anti-NKp30 over isotype control. However, KHYG-1 was less effective at inhibiting OCI/AML5 colony formation alone, but this could be enhanced three-fold by pretreatment with anti-NKp30 antibody. The inhibition of colonies indicates a cytotoxic or cytostatic effect on clonogenic cells within the cell line populations that represent leukemic stem and progenitor cells. Therefore, this provided evidence that reverse ADCC facilitated cytotoxicity against leukemic stem cells.

To evaluate the impact of NK cell line therapy *in vivo,* we established an OCI/AML5 xenograft model in NSG mice. OCI/AML5 was derived from a patient with M4 leukemia, and highly expresses CD33, which is useful for tracking *in vivo* (Wang, Koistinen et al. 1991). We confirmed injection of OCI/AML5 *iv* led to leukemia with splenomegaly and bone marrow engraftment. However, *ip* injection tended to cause progressive malignant ascites over a longer timeframe, rather than classic leukemia.

To determine the effect of *in vitro* cytotoxicity on *in vivo* proliferation, we incubated OCI/AML5 with or without iKHYG-1 (+/-anti-NKp30 pretreatment) and injected the cells *ip.* We utilized this injection route instead of *iv,* because of the high cell load (2x10⁶ OCI/AML5 + 20x10⁶ viable iKHYG-1 + ∼3x10⁶ non-viable iKHYG-1) and relatively larger KHYG-1 cells, which might have caused pulmonary stress to the mice if injected via tail vein. Co-incubation with iKHYG-1 had no impact, while anti-NKp30 pretreated iKHYG-1 improved median survival by 10 days.

We subsequently utilized the *iv* injection OCI/AML5 NSG xenograft model to test therapeutic efficacy of iKHYG-1 with or without anti-NKp30 pretreatment. Unexpectedly, iKHYG-1 was able to improve the median survival by 35 days, despite its poor cytotoxicity in the CRA against bulk OCI/AML5. However, KHYG-1 had three-fold better cytotoxicity against clonogenic OCI/AML5 than bulk OCI/AML5, as determined by the CRA, providing some basis for this finding. This is the first demonstration of efficacy by KHYG-1 in an *in vivo* cancer model and confirms that the irradiated cells can persist and reduce tumour burden. This is also the first evidence that the irradiated cells can function *in vivo.* We then used a primary AML xenograft model to test iKHYG-1 and anti-NKp30 iKHYG-1, demonstrating lack of efficacy of iKHYG-1, but a trend to improved survival in the anti-NKp30 iKHYG-1 treated group.

In summary, we demonstrated that NK-92 and KHYG-1 have cytotoxicity against a broad range of leukemic targets that can be enhanced several-fold by anti-NKp30 and anti-NKp44 antibodies. For KHYG-1, cancer cell killing was achieved via R-ADCC via interaction of antibody coated effectors with FcγRII (CD32) on the target cells. Furthermore, antibodies to NK cell surface markers enhanced cytotoxicity of KHYG-1 against clonogenic leukemic cells (cancer stem cells) and reduced *in vivo* proliferation of leukemia. These results are relevant to treatment of cancers via R-ADCC using the patient's own NK cells instead of an NK cell line, which approach offers an alternative and suitably an improved therapy: the patient's own NK cells are less likely to provoke adverse reactions and treatment may be simpler as an exogenous cell line need not be provided and administered.

We have therefore devised protocols for determining whether cancers are suitable for RADCC-based treatment and for treating cancer via RADCC using the patient's own NK cells (both endogenous and *ex vivo* activated autologous NK cells).

The invention hence provides methods, uses and compositions for treatment for tumours using antibodies, and the patient's own NK cells or both in combination via R-ADCC.

### Additional References

Notter, M., W. D. Ludwig, et al. (1993). "Selective targeting of human lymphokine-activated killer cells by CD3 monoclonal antibody against the interferon-inducible high-affinity Fc gamma Rl receptor (CD64) on autologous acute myeloid leukemic blast cells." Blood 82(10): 3113-3124.
Pende, D., S. Parolini, et al. (1999). "Identification and molecular characterization of NKp30, a novel triggering receptor involved in natural cytotoxicity mediated by human natural killer cells." Journal of Experimental Medicine 190(10): 1505-1516.
Saxena, R. K., Q. B. Saxena, et al. (1982). "Identity of effector cells participating in the reverse antibody-dependent cell-mediated cytotoxicity." Immunology 46(2): 459-464.
Lee, H. W. et al. (2014). "Evaluation of therapeutic effects of natural killer (NK) cell-based immunotherapy in mice using in vivo apoptosis bioimaging with a caspace-3 sensor." The FASEB Journal 28(7):2932-2941.
Jing, Y. et al. (2015). "Identification of an ADAM17 cleavage region in human CD16 (Fc[gamma]RIII) and the engineering of a non-cleavable version of the receptor in NK cells." PLOS One 10(3):e0121788.
Ferlazzo, G. et al. (2002). "Human dendritic cells activate resting natural killer (NK) cells and are recognized via the NKp30 receptor by anctivated NK cells." 195(3):343-351.
Williams, B.A. (2010). "Clonogenic assays measure leukemia stem cell killing not dectectable by chromium release and flow cytometric cytotoxicity assays." Cytotherapy 12(7):951-960.
Arnon, T.I. et al. (2008). "Harnessing soluble NK cell killer receptors for the generation of novel cancer immune therapy." PLOS One 3(5):e2150.
Williams, B. (2010). "NK-92 preferentially targets acute myeloid leukemia stem cells." Blood 116(21):1745.
Suck, G. et al. (2006). "Irradiated KHYG-1 retains cytotoxicity: potential for adoptive immunotherapy with a natural killer cell line." International Journal of Radiation Biology 82(5):355-361.
Swift, B. et al. (2012). "Natural killer cell lines preferentially kill clonogenic multiple myeloma cells and decrease myeloma engraftment in a bioluminescent xenograft mouse model." Haematologica-The Hematology Journal 97(7):1020-1028.
Kobayashi, E. et al. (2014). "Antibody-dependent cellular cytotoxicity and cytokine/chemokine secretion by KHYG-1 cells stably expressing Fc[gamma]RIIIA." Immunology Letters 161(1):59-64.
Grier, J.T. et al. (2012). "Human immunodeficiency-causing mutation defines CD16 in spontaneous NK cell cytotoxicity." Journal of Clinical Investigation 122(10):3769-3780.
Deng, X. (2014). "Ex vivo-expanded natural killer cells kill cancer cells more effectively than ex vivo-expanded [gamma] [delta] T cells." International Immunopharmacology 22(2):486-491.
Muller, T. et al. (2014). "Allogeneic and xenogeneic anti-tumor effect of callithrix jacchus natural killer cells is dependent on KNp30 and B7-H6 interaction. Journal of Biological Regulators and Homeostatic Agents 28(2):183-193.
Swift, B. et al. (2011). "Efficacy of NK-92 against human multiple myeloma in a NOD/SCID gamma null mouse model." Blood 118(21):807.
Lagrue, K. et al. (2015). "Lenalidomide augments actin remodeling and lowers NK-cell activation thresholds." Blood 126(1):50-60.
Liu, Y-C. et al. (2014). "Profile of elotuzumab and its potential in the treatment of multiple myeloma." Journal of Blood and Lymphatic Cancer 2014(4):15-27.
Barber, N. et al. (2008). "All-trans retinoic acid induces different immunophenotypic changes on human HL60 and NB4 myeloid leukaemias." Leukemia Research 32(2):315-322.

## Claims

1. A combination of an antibody and a chemotherapy agent, for use in treating a cancer expressing Fc receptors in a patient via reverse antibody-dependent cell-mediated cytotoxicity (R-ADCC), wherein
the antibody binds an antigen on the surface of an endogenous or autologous natural killer (NK) cell;
the antibody further binds an Fc receptor on a cell of the cancer; and
the agent is not an antibody.

2. A combination for use according to claim 1, wherein the treating comprises upregulating Fc receptor expression on the cancer, e.g. by administration of the chemotherapy agent.

3. A combination for use according to any preceding claim, wherein the chemotherapy agent is an alkylating agent.

4. A combination for use according to claim 3, wherein the alkylating agent is selected from a nitrogen mustard, a nitrosourea, an alkyl sulfonate, a triazine or an ethylenimine.

5. A combination for use according to claim 4, wherein the nitrogen mustard is selected from bendamustine, chlorambucil, cyclophosphamide, ifosfamide, mechlorethamine or melphalan.

6. A combination for use according to either claim 4 or 5, wherein the nitrogen mustard is cyclophosphamide.

7. A combination for use according to any preceding claim, further comprising an agent capable of activating endogenous or autologous NK cells.

8. A combination for use according to any preceding claim, wherein the autologous NK cell is expanded and activated ex vivo and administered to the patient in combination with the antibody and chemotherapy agent.

9. A combination for use according to claim 8, wherein the treating comprises administering the antibody bound to the NK cell.

10. A combination for use according to any of claims 7 to 9, wherein the activating agent is a thalidomide derivative, e.g. lenalidomide.

11. A combination for use according to any preceding claim, wherein the cancer does not express an antigen for the antibody.

12. A combination for use according to any preceding claim, wherein the cancer is a blood cancer selected from acute lymphocytic leukemia (ALL), acute myeloid leukemia (AML), chronic lymphocytic leukemia (CLL), chronic myeloid leukemia (CML), Hodgkin's lymphoma, non-Hodgkin's lymphoma, including T-cell lymphomas and B-cell lymphomas, asymptomatic myeloma, multiple myeloma (MM), smoldering multiple myeloma (SMM), active myeloma and light chain myeloma.

13. A combination for use according to claim 12, wherein the blood cancer is AML.

14. A combination for use according to claim 12, wherein the blood cancer is MM.

15. A composition for the treatment of cancer, comprising the combination of an antibody and a chemotherapy agent according to any preceding claim.
